(19) Europäisches Patentamt
European Patent Office
Office européen des brevets

(11) Veröffentlichungsnummer: **0 338 228**
**A2**

(12) **EUROPÄISCHE PATENTANMELDUNG**

(21) Anmeldenummer: **89103966.1**

(22) Anmeldetag: **07.03.89**

(51) Int. Cl.⁴: **A61K 31/47 , A61K 31/55 , C07D 215/22**

Die Bezeichnung der Erfindung wurde geändert (Richtlinien für die Prüfung im EPA, A-III, 7.3).

(30) Priorität: **11.03.88 DE 3808136**

(43) Veröffentlichungstag der Anmeldung: **25.10.89 Patentblatt 89/43**

(84) Benannte Vertragsstaaten: **AT BE CH DE ES FR GB GR IT LI LU NL SE**

(71) Anmelder: **Dr. Karl Thomae GmbH**
**Postfach 1755**
**D-7950 Biberach 1(DE)**

(72) Erfinder: **Müller, Erich, Dr. Dipl.-Chem.**
**Talfeldstrasse 34**
**D-7950 Biberach 1(DE)**
Erfinder: **Nickl, Josef, Dr. Dipl.-Chem.**

**(verstorben)(DE)**
Erfinder: **Heckel, Armin, Dr. Dipl.-Chem.**
**Lampartenweg 1**
**D-7950 Biberach 1(DE)**
Erfinder: **Engelhardt, Günther, Prof. Dr.**
**Unterer Bühl 18**
**D-7950 Biberach 1(DE)**

(54) **Verwendung von Chinolin-2,5-dione in einem Arzneimittel mit einer analgetischen, antipyretischen und/oder einer antiphlogistischen Wirkung.**

(57) Die vorliegende Erfindung betrifft Arzneimittel, enthaltend Chinolin-2,5-dione der Formel

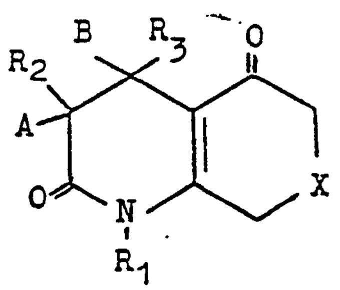

,(I)

in der

A, B, $R_1$ bis $R_3$ und X wie in den Ansprüchen 1 bis 6 definiert sind, sowie neue Chinolin-2,5-dione gemäß den Ansprüchen 10 bis 14, welche wertvolle pharmakologische Eigenschaften aufweisen, insbesondere eine analgetische, antipyretische und/oder antiphlogistische Wirkung, neue Zwischenprodukte und Verfahren zu ihrer Herstellung.

EP 0 338 228 A2

**Arzneimittel, enthaltend Chinolin-2,5-dione, neue Chinolin-2,5-dione und Verfahren zu ihrer Herstellung**

In der Literatur werden bereits Chinolin-2,5-dione ohne irgendwelche Angaben über ihre pharmakologischen Eigenschaften beschrieben (siehe Arch. Pharm. 308, 588-594 (1975), J. Het. Chem. 22, 1503-1509 (1985), J. Org. Chem. 33, 1089-1092 (1968), Eur. J. Med. Chem. 14, 499-506 (1979), Chem. Ber. 103, 2403-2410 (1970), C. A. 67, 99689, Bull. Soc. Chim. Belge 88, 671-676 (1979), J. Org. Chem. 46, 3719-3721 (1981), Tetrahedron Letters 1965, 2441-2444, Tetrahedron Letters 1967, 2563-2566 und J. Chem. Soc., Perkin Trans. 1 1984, 287-290).

Außerdem werden in der japanischen Offenlegungsschrift 76/32.568 unter anderem in 1-Stellung durch eine Ethyl-oder Allylgruppe substituierte 5-(3-Amino-2-hydroxy-propoxy)-3,4-dihydro-carbostyrilderivate beschrieben, wobei die zu ihrer Herstellung erforderlichen 1-Ethyl- und 1-Allyl-5-oxo-hexahydro-carbostyrile expressis verbis nicht beschrieben werden.

Es wurde nun gefunden, daß die Chinolin-2,5-dione der Formel

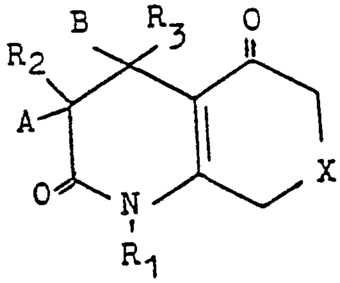

,(I)

und deren optisch aktive Antipoden, sofern diese ein optisch aktives Kohlenstoffatom enthalten, wertvolle pharmakologische Eigenschaften aufweisen, insbesondere eine analgetische, antipyretische und/oder antiphlogistische Wirkung.

In der obigen Formel I bedeutet

A und B jeweils ein Wasserstoffatom oder zusammen eine Kohlenstoff-Kohlenstoff-Bindung,

$R_1$ ein Wasserstoffatom, eine gegebenenfalls durch eine Phenyl-, Fluorphenyl-, Chlorphenyl- oder Bromphenylgruppe, durch eine Carboxygruppe oder durch eine Alkoxycarbonylgruppe mit insgesamt 2 bis 4 Kohlenstoffatomen substituierte Alkylgruppe mit 1 bis 3 Kohlenstoffatomen, eine Alkylgruppe mit 4 bis 6 Kohlenstoffatomen, eine Cycloalkylgruppe mit 3 bis 6 Kohlenstoffatomen, eine Alkenyl- oder Alkinylgruppe mit jeweils 3 bis 5 Kohlenstoffatomen, eine in 2- oder 3-Stellung durch eine Hydroxy-, Alkoxy- oder Alkylmercaptogruppe substituierte Alkylgruppe mit 2 oder 3 Kohlenstoffatomen, in welcher der Alkoxy- oder Alkylmercaptosubstituent jeweils 1 bis 3 Kohlenstoffatome enthalten kann, eine gegebenenfalls durch ein Halogenatom, durch eine Alkyl- oder Alkoxygruppe mit jeweils 1 bis 3 Kohlenstoffatome im Alkylteil substituierte Phenylgruppe oder eine Tetrahydrofurforylgruppe,

$R_2$ ein Wasserstoffatom oder eine Alkylgruppe mit 1 bis 3 Kohlenstoffatomen,

$R_3$ ein Wasserstoffatom, eine Trifluormethyl-, Phenyl- oder Alkylgruppe mit 1 bis 3 Kohlenstoffatomen oder

$R_2$ und $R_3$ zusammen eine n-Alkylengruppe mit 3 bis 5 Kohlenstoffatomen und

X eine gegebenenfalls durch eine oder zwei Alkylgruppen mit jeweils 1 bis 3 Kohlenstoffatomen substituierte Methylengruppe.

Hierbei sind diejenigen Chinolin-2,5-dione neu, in denen,

wenn A und B zusammen eine weitere Kohlenstoff-Kohlenstoff-Bindung darstellen,

$R_2$ eine Alkylgruppe mit 1 bis 3 Kohlenstoffatomen darstellt und $R_1$, $R_3$ und X wie eingangs definiert sind oder

$R_1$ mit Ausnahme des Wasserstoffatoms, der Methyl- und Ethylgruppe die für $R_1$ eingangs erwähnten Bedeutungen besitzt und $R_2$, $R_3$ und X wie eingangs definiert sind oder,

wenn A und B jeweils ein Wasserstoffatom darstellen,

$R_1$ mit Ausnahme des Wasserstoffatoms, der Methylgruppe und der Benzylgruppe die für $R_1$ eingangs erwähnten Bedeutungen besitzt und $R_2$, $R_3$ und X wie eingangs definiert sind oder

$R_2$ eine Alkylgruppe mit 2 oder 3 Kohlenstoffatomen darstellt und $R_1$, $R_3$ und X wie eingangs definiert sind.

Gegenstand der vorliegenden Erfindung sind somit die neuen Arzneimittel, enthaltend eine Verbindung der Formel I, welche zur Bekämpfung von Schmerzzuständen, von Fieber und Entzündungen sowie zur Bekämpfung der Symptomatik von Erkältungskrankheiten geeignet sind, die neuen Chinolin-2,5-dione der

vorstehenden Formel I und Verfahren zu ihrer Herstellung.

Für die bei der Definition der Reste $R_1$, $R_2$, $R_3$ und X eingangs erwähnten Bedeutungen kommt beispielsweise

für $R_1$ die Bedeutung des Wasserstoffatoms, der Methyl-, Ethyl-, n-Propyl-, Isopropyl-, n-Butyl-, Isobutyl-, n-Pentyl-, n-Hexyl-, Cyclopropyl-, Cyclobutyl-, Cyclopentyl-, Cyclohexyl-, Allyl-, n-But-2-enyl-, n-But-3-enyl-, n-Pent- 2-enyl-, n-Pent-3-enyl, Propargyl-, n-But-2-inyl-, n-But-3-inyl-, n-Pent-2-inyl-, n-Pent-3-inyl-, 2-Methyl-n-but-2-enyl-, Benzyl-, Fluorbenzyl-, Chlorbenzyl-, Brombenzyl-, 1-Phenyl-ethyl-, 2-Phenyl-ethyl-, 3-Phenyl-propyl-, 2-(Chlorphenyl)-ethyl-, Carboxymethyl-, 1-Carboxyethyl-, 2-Carboxyethyl-, 1-Carboxy-n-propyl-, 2-Carboxy-n-propyl-, 3-Carboxypropyl-, Methoxycarbonylmethyl-, 1-Ethoxycarbonyl-ethyl-, 2-Isopropoxycarbonyl-ethyl-, 3-Ethoxycarbonyl-n-propyl-, 2-Hydroxy-ethyl-, 2-Hydroxy-n-propyl-, 2-Hydroxy-1-methylethyl-, 2-Methoxy-ethyl-, 3-Ethoxy-n-propyl-, 2-n-Propoxy-1-methyl-ethyl-, 2-Methylmercapto-ethyl-, 2-Ethylmercapto-n-propyl-, 2-Isopropylmercapto-1-methyl-ethyl-, 3-Methyl-mercapto-n-propyl-, Phenyl-, Fluorphenyl-, Chlorphenyl-, Bromphenyl-, Hydroxyphenyl-, Methylphenyl-, Ethylphenyl-, Isopropyl-phenyl-, Methoxyphenyl-, Ethoxyphenyl-, n-Propoxy-phenyl-oder Tetrahydrofurforylgruppe,

für $R_2$ die des Wasserstoffatoms, der Methyl-, Ethyl-, n-Propyl- oder Isopropylgruppe,

für $R_3$ die des Wasserstoffatoms, der Methyl-, Ethyl-, n-Propyl-, Isopropyl-, Trifluormethyl- oder Phenylgruppe oder

für $R_2$ und $R_3$ zusammen die n-Propylen-, n-Butylen- oder n-Pentylengruppe und

für X die der Methylen-, Methyl-methylen-, Dimethyl-methylen-, Methyl-ethyl-methylen-, Diethyl-methylen- oder n-Propyl-methylengruppe in Betracht.

Beispielsweise seien noch folgende Verbindungen genannt, die unter den Schutzumfang der vorliegenden Erfindung fallen, aber in den Beispielen nicht explicit beschrieben werden:

1-Isopropyl-3,4,7,8-tetrahydro-2,5(1H,6H)-chinolindion,

1-n-Butyl-3,4,7,8-tetrahydro-2,5(1H,6H)-chinolindion,

1-Allyl-3,4,7,8-tetrahydro-2,5(1H,6H)-chinolindion und

1-Propargyl-3,4,7,8-tetrahydro-2,5(1H,6H)-chinolindion.

Bevorzugte Verbindungen der eingangs erwähnten Formel I sind jedoch diejenigen, in denen

A und B jeweils ein Wasserstoffatom oder zusammen eine weitere Kohlenstoff-Kohlenstoff-Bindung,

$R_1$ ein Wasserstoffatom, eine Alkylgruppe mit 1 bis 4 Kohlenstoffatomen, eine in 2-Stellung durch eine Hydroxy-, Methoxy-, Methylmercapto- oder Phenylgruppe substituierte Ethylgruppe, eine gegebenenfalls durch ein Fluor-, Chlor-oder Bromatom, eine Methyl- oder Methoxygruppe substituierte Phenylgruppe, eine Cyclohexyl-, 3-Methoxy-propyl-, Allyl-, Propargyl-, Carboxymethyl-, Methoxycarbonylmethyl-, Benzyl-, Chlorbenzyl- oder Tetrahydrofurforylgruppe,

$R_2$ ein Wasserstoffatom oder eine Methylgruppe,

$R_3$ ein Wasserstoffatom, eine Methyl-, Ethyl-, Trifluormethyl- oder Phenylgruppe oder

$R_2$ und $R_3$ zusammen eine n-Propylen- oder n-Butylengruppe und

X eine Methylen-, Methyl-methylen- oder Dimethyl-methylengruppe darstellen,

wobei diejenigen Chinolin-2,5-dione neu sind, in denen,

wenn A und B zusammen eine weitere Kohlenstoff-Kohlenstoff-Bindung darstellen,

$R_2$ eine Methylgruppe darstellt und $R_1$, $R_3$ und X wie vorstehend definiert sind oder

$R_1$ mit Ausnahme des Wasserstoffatoms, der Methyl- und Ethylgruppe die für $R_1$ vorstehend erwähnten Bedeutungen besitzt und $R_2$, $R_3$ und X wie vorstehend definiert sind oder,

wenn A und B jeweils ein Wasserstoffatom darstellen,

$R_1$ mit Ausnahme des Wasserstoffatoms, der Methylgruppe, der Ethylgruppe, der Allylgruppe und der Benzylgruppe die für $R_1$ vorstehend erwähnten Bedeutungen besitzt und $R_2$, $R_3$ und X wie vorstehend definiert sind.

Besonders bevorzugte Verbindungen der eingangs erwähnten Formel I sind jedoch diejenigen, in denen

A und B jeweils ein Wasserstoffatom oder zusammen eine weitere Kohlenstoff-Kohlenstoff-Bindung,

$R_1$ ein Wasserstoffatom, eine Alkylgruppe mit 1 bis 4 Kohlenstoffatomen oder eine in 2-Stellung durch eine Hydroxy-, Methoxy- oder Methylmercaptogruppe substituierte Ethylgruppe,

$R_2$ ein Wasserstoffatom oder eine Methylgruppe und $R_3$ ein Wasserstoffatom oder

$R_2$ ein Wasserstoffatom und $R_3$ eine Methyl- oder Ethylgruppe und

X eine Methylengruppe bedeuten, wobei diejenigen Chinolin-2,5-dione neu sind, in denen,

wenn A und B zusammen eine weitere Kohlenstoff-Kohlenstoff-Bindung darstellen,

$R_2$ eine Methylgruppe und

$R_3$ ein Wasserstoffatom bedeuten und

$R_1$ und X wie vorstehend erwähnt definiert sind oder

$R_1$ mit Ausnahme des Wasserstoffatoms, der Methyl- und Ethylgruppe die für $R_1$ vorstehend erwähnten

Bedeutungen besitzt und $R_2$, $R_3$ und X wie vorstehend erwähnt definiert sind oder,
wenn A und B jeweils ein Wasserstoffatom darstellen sowie
$R_1$ mit Ausnahme des Wasserstoffatoms, der Methylgruppe und der Ethylgruppe die für $R_1$ vorstehend erwähnten Bedeutungen besitzt und $R_2$, $R_3$ und X wie vorstehend erwähnt definiert sind.

Die vorstehend erwähnten neuen Chinolin-2,5-dione erhält man erfindungsgemäß nach folgenden Verfahren:

a) Umsetzung einer Verbindung der Formel

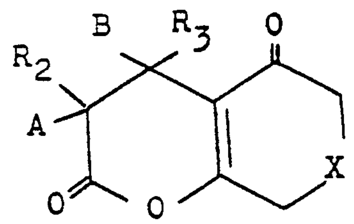
,(II)

in der
A, B, $R_2$, $R_3$ und X wie eingangs definiert sind, mit einem Amin der Formel

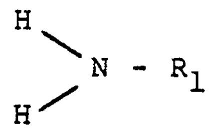
,(III)

in der
$R_1$ wie eingangs definiert ist.
Die Umsetzung wird vorzugsweise in einem Lösungsmittel wie Methanol, Ethanol, Isopropanol, Tetrahydrofuran, Dioxan, Me thylenchlorid oder Benzol oder auch in einem Überschuß des als Lösungsmittel eingesetzten Amins der Formel III zweckmäßigerweise bei Temperaturen zwischen -30 und 180 °C, vorzugsweise jedoch bei Temperaturen zwischen 15 und 50 °C, durchgeführt. Die Umsetzung kann jedoch auch ohne Lösungsmittel durchgeführt werden.

b) Umsetzung einer Verbindung der Formel

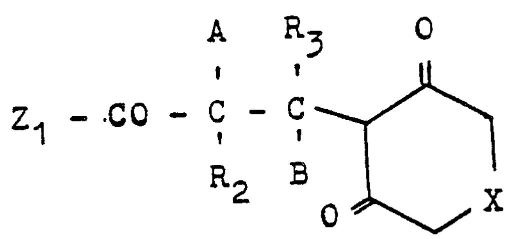
,IV

in der
A, B, $R_2$, $R_3$ und X wie eingangs definiert sind und
$Z_1$ eine nucleophile Austrittsgruppe wie eine Hydroxygruppe, eine Alkoxygruppe mit 1 bis 3 Kohlenstoffatomen oder ein Halogenatom darstellt, mit einem Amin der Formel

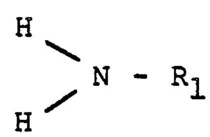
,(III)

in der
$R_1$ wie eingangs definiert ist.
Die Umsetzung wird vorzugsweise in einem Lösungsmittel wie Methanol, Ethanol, Isopropanol, Tetrahydrofuran, Dioxan, Methylenchlorid oder Benzol oder auch in einem Überschuß des als Lösungsmittel eingesetzten Amins der Formel III zweckmäßigerweise bei Temperaturen zwischen -30 und 200 °C, vorzugsweise jedoch bei Temperaturen zwischen 140 und 180 °C, durchgeführt. Die Umsetzung kann jedoch auch ohne Lösungsmittel durchgeführt werden.

c) Zur Herstellung von Verbindungen der Formel I, in der $R_2$ ein Wasserstoffatom darstellt:
Decarboxylierung einer Verbindung der Formel

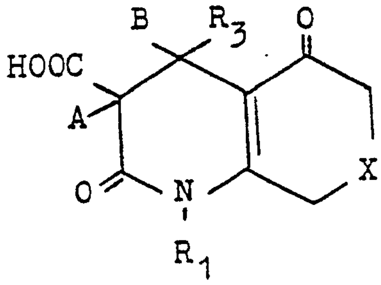

, (V)

in der
A, B, $R_1$, $R_3$ und X wie eingangs definiert sind.
Die Decarboxylierung wird vorzugsweise in einem hochsiedenden Lösungsmittel wie Chinolin, Collidin, Dimethylsulfoxid, Diphenyl, Tetralin, Dekalin, o-Dichlorbenzol, Ethylenglycol oder 2-n-Butoxy-ethanol und gegebenenfalls in Gegenwart eines Reaktionsbeschleunigers wie Kupferpulver bei erhöhten Temperaturen, z.B. bei Temperaturen oberhalb 80 °C, vorzugsweise jedoch bei Temperaturen zwischen 100 und 200 °C, durchgeführt. Die Umsetzung kann jedoch auch ohne Lösungsmittel durchgeführt werden.
Besonders vorteilhaft wird die Umsetzung jedoch in der Weise durchgeführt, daß eine Verbindung der allgemeinen Formel V im Reaktionsgemisch hergestellt wird, beispielsweise durch Erhitzen eines entsprechenden Esters wie des Methyl-, Ethyl-oder Isopropylesters in einem geeigneten Lösungsmittel wie Dimethylsulfoxid in Gegenwart eines Alkalimetallhalogenids, vorzugsweise von Lithiumchlorid oder Natriumchlorid, und in Gegenwart von Wasser. Die erforderliche Menge an eingesetztem Wasser beträgt hierbei zweckmäßigerweise die 2- bis 6-fache äquimolare Menge, vorzugsweise jedoch die 3- bis 4-fach äquimolare Menge, bezogen auf den eingesetzten Ester.
Eine eingesetzte Verbindung der Formel V kann auch im Reaktionsgemisch hergestellt werden.

d) Zur Herstellung von Verbindung der Formel I, in der $R_1$ mit Ausnahme des Wasserstoffatoms wie eingangs definiert ist:
Umsetzung einer Verbindung der Formel

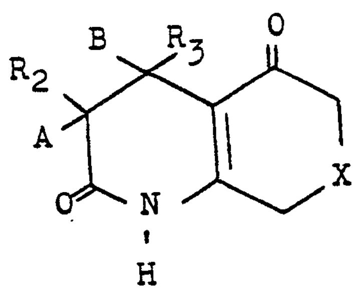

, (VI)

in der
A, B, $R_2$, $R_3$ und X wie eingangs definiert sind, mit einer Verbindung der Formel

$Y - R_1'$ ,(VII)

in der
$R_1'$ mit Ausnahme des Wasserstoffatoms die für $R_1$ eingangs erwähnten Bedeutungen besitzt und
Y eine nukleophile Austrittsgruppe wie ein Halogenatom, z.B. ein Chlor-, Brom- oder Jodatom, oder einen Sulfonyloxyrest, z.B. die Methansulfonyloxy- oder p-Toluolsulfonyloxygruppe, oder
Y zusammen mit einem β-Wasserstoffatom einer Alkylgruppe mit 2 bis 6 Kohlenstoffatomen des Restes $R_1'$ ein Sauerstoffatom darstellt.
Die Alkylierung wird in einem geeigneten Lösungsmittel wie Methanol, Ethanol, Diethylether, Aceton, Methylenchlorid, Tetrahydrofuran, Dioxan, Dimethylformamid oder Dimethylsulfoxid, vorzugsweise jedoch in

einem aprotischen Lösungsmit tel wie Aceton, Dimethylformamid oder Dimethylsulfoxid, in Gegenwart einer anorganischen Base wie Kaliumcarbonat, Natriumhydroxid, Natriumhydrid oder Kaliumhydroxid mit einem geeigneten Alkylierungsmittel wie Methyljodid, Dimethylsulfat, Ethylbromid, Diethylsulfat, Benzylchlorid, n-Propylbromid, Isopropylbromid, 2-Hydroxy-ethylbromid, Ethylenoxid oder n-Propylen-2,3-oxid bei Temperaturen zwischen 0 und 100° C, vorzugsweise jedoch bei Temperaturen zwischen 10 und 50° C, durchgeführt.

e) Zur Herstellung von Verbindungen der Formel I, in der A und B je ein Wasserstoffatom darstellen: Umsetzung eines Enaminoketons der Formel

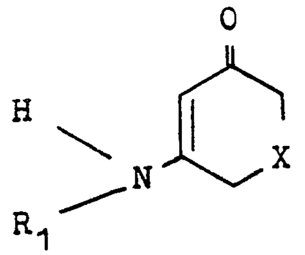

,(VIII)

in der

R· und X wie eingangs definiert sind, mit einer Verbindung der Formel

$$R_3 - CH = \overset{R_2}{\overset{|}{C}} - CO - Z_2$$

,(IX)

in der

$R_2$ und $R_3$ wie eingangs definiert sind und

$Z_2$ eine nukleophile Austrittsgruppe wie eine Hydroxygruppe, eine Alkoxygruppe mit 1 bis 3 Kohlenstoffatomen oder ein Halogenatom darstellt.

Die Umsetzung wird zweckmäßigerweise in einem hochsiedenden Lösungsmittel wie Tetralin, Dekalin, Diphenyl oder o-Dichlorbenzol, vorzugsweise jedoch ohne Lösungsmittel bei er höhten Temperaturen, z.B. bei Temperaturen zwischen 100 und 250° C, vorzugsweise jedoch bei Temperaturen zwischen 120 und 180° C, durchgeführt. Besonders vorteilhaft wird jedoch die Umsetzung in einem Druckgefäß durchgeführt.

Wie bereits eingangs erwähnt, können die neuen Verbindungen in Form ihrer Enantiomeren, Enantiomerengemische oder Racemate, oder sofern sie 2 asymmetrische Kohlenstoffatome enthalten, auch in Form ihrer Diastereomeren bzw. Diastereomerengemische vorliegen.

So lassen sich die erhaltenen Verbindungen der allgemeinen Formel I, welche nur ein optisch aktives Zentrum enthalten, nach an sich bekannten Methoden (siehe Allinger N. L. und Elich W. L. in "Topics in Stereochemistry", Vol. 6, Wiley Interscience, 1971) in ihre optischen Antipoden auftrennen, z.B. durch Umkristallisieren aus einem optisch aktiven Lösungsmittel.

Desweiteren lassen sich die erhaltenen Verbindungen der allgemeinen Formel I mit 2 asymmetrischen Kohlenstoffatomen auf Grund ihrer physikalischen-chemischen Unterschiede nach an sich bekannten Methoden, z.B. durch Chromatographie und/oder fraktionierte Kristallisation, in ihre Diastereomeren auftrennen. Ein so erhaltenes Enantiomerenpaar läßt sich anschließend in seine optischen Antipoden, wie oben beschrieben, auftrennen.

Die als Ausgangsstoffe verwendeten Verbindungen der Formeln II bis IX erhält man nach literaturbekannten Verfahren.

So erhält man eine Verbindung der Formel II oder IV durch Reaktion eines entsprechenden Cyclohexan-1,3-dions mit einem entsprechenden Carbonsäurederivat,

eine Verbindung der Formel V, welche teilweise neu sind, durch Umsetzung eines entsprechenden 2-Amino-methylen-cyclohexan-1,3-dions mit einem Cyanessigester und anschließender Verseifung oder durch Umsetzung mit einem entsprechenden Cyclohexan-1,3-dion mit einem Alkoxymethylencyanessigester und anschließender Verseifung und eine Verbindung der Formel VI durch Ringschluß eines entsprechenden 1-Amino-cyclohexen-3-ons mit einem entsprechenden $\alpha$, $\beta$-ungesättigten Carbonsäure-derivat.

Eine stereoselektive Ausgangsverbindung der Formel V, die nur ein optisch aktives Zentrum in 4-Position enthält, läßt sich auch analog der von Enders et al. in Tetrahedron Letters <u>28</u>, 3795-3798 (1987)

beschriebenen Methode herstellen. Hierzu wird beispielsweise (S)-1-Amino-2-methoxymethyl-pyrrolidin mit Dimedon oder Cyclohexan-1,3-dion in das entsprechende (S)-Hydrazonderivat übergeführt, welches anschließend nach Metallierung, z. B. mit n-Butyllithium bei -78° C, mit einem entsprechenden Benzylidenmalonsäureester umgesetzt wird. Das so erhaltene (SR)-2-[(2-Bis(alkoxycarbonyl)-1-phenyl)-ethyl]-3-[(2-methoxymethyl)-pyrrolidin-1-yl]-en-amino-cyclohexan-3-on wird zu einem entsprechenden Chinolindion ringgeschlossen, welches mittels saurer Reduktion, z. B. mit Zink/Eisessig, und gleichzeitiger Verseifung in die gewünschte Verbindung der Formel V übergeführt wird, wobei diese nicht isoliert wird.

Eine weiterer Gegenstand der vorliegenden Erfindung sind die neuen Chinolindione der Formel

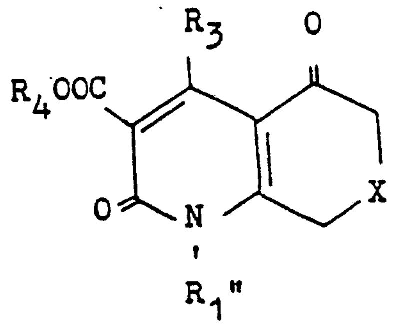

, (X)

in der

$R_3$ und X wie eingangs definiert sind,

$R_1''$ ein Wasserstoffatom, eine gegebenenfalls durch eine Phenyl-, Fluorphenyl-, Chlorphenyl- oder Bromphenylgruppe, durch eine Carboxygruppe oder durch eine Alkoxycarbonylgruppe mit insgesamt 2 bis 4 Kohlenstoffatomen substituierte Alkylgruppe mit 1 bis 3 Kohlenstoffatomen, eine Alkylgruppe mit 4 bis 6 Kohlenstoffatomen, wie Cycloalkylgruppe mit 3 bis 6 Kohlenstoffatomen, eine Alkenyl- oder Alkinylgruppe mit jeweils 3 bis 5 Kohlenstoffatomen, eine in 2- oder 3-Stellung durch eine Hydroxy-, Alkoxy- oder Alkylmercaptogruppe substituierte Alkylgruppe mit 2 oder 3 Kohlenstoffatomen, in welcher der Alkoxy- oder Alkylmercaptosubstituent jeweils 1 bis 3 Kohlenstoffatome enthalten kann, oder eine Tetrahydrofurforylgruppe und

$R_4$ eine Alkylgruppe mit 1 bis 5 Kohlenstoffatomen oder

$R_1''$ mit Ausnahme des Wasserstoffatoms die für $R_1''$ vorstehend erwähnten Bedeutungen besitzt und

$R_4$ ein Wasserstoffatom bedeuten, welches sich direkt oder nach ihrer Überführung in die entsprechende Carbonsäure in eine entsprechende Verbindung der Formel I, in der $R_1''$,

$R_3$ und X wie eingangs definiert sind und $R_2$ ein Wasserstoffatom bedeutet, überführen lassen.

Erfindungsgemäß erhält man die neuen Verbindungen durch Umsetzung eines Cyclohexan-1,3-dions der Formel

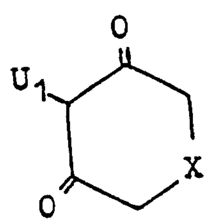

, (XI)

in der

X wie eingangs definiert ist, mit einem Cyanessigester der Formel

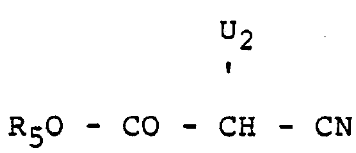

, (XII)

in der

$U_1$ ein Wasserstoffatom und $U_2$ zusammen mit dem Wasserstoffatom der benachbarten CH-Gruppe eine

Gruppe der Formel

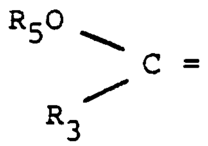 oder

$U_1$ zusammen mit dem Wasserstoffatom der benachbarten CH-Gruppe eine Gruppe der Formel

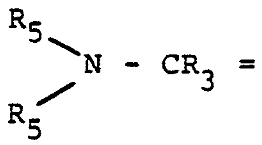 und $U_2$ ein Wasserstoffatom

bedeuten, wobei $R_3$ wie eingangs definiert ist und $R_5$, die gleich oder verschieden sein können, jeweils eine Alkylgruppe mit 1 bis 5 Kohlenstoffatomen darstellen,
Umsetzung eines so erhaltenen Tetrahydro-cumarinons der Formel

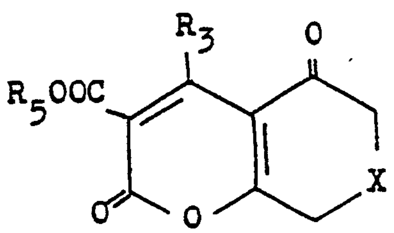 ,(XIII)

in der
$R_3$ und X wie eingangs definiert sind und
$R_5$ wie vorstehend erwähnt definiert ist, mit einem Amin der Formel

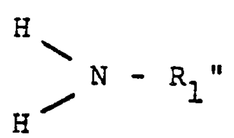 ,(IIIa)

in der
$R_1''$ wie vorstehend definiert ist, und gewünschtenfalls anschließende Hydrolyse eines so erhaltenen Esters.

Die Ringschlußreaktion wird vorzugsweise in einem Lösungsmittel wie Dimethylformamid, Dimethylsulfoxid, Dioxan oder Chloroform gegebenenfalls in Gegenwart einer Base wie Natriumethylat, Kaliumhydroxid, Kalium-tert.butylat oder Natriumhydrid bei Temperaturen zwischen -20 und 50°C, vorzugsweise jedoch bei Raumtemperatur, durchgeführt.

Die anschließende Umsetzung mit einem Amin der Formel III wird zweckmäßigerweise in einem Lösungsmittel wie Ethanol, Dimethylformamid, Dimethylsulfoxid, Dioxan, Chloroform oder auch in dem eingesetzten Amin der Formel III als Lösungsmittel bei Temperaturen zwischen 0 und 50°C, vorzugsweise bei Raumtemperatur, durchgeführt.

Die anschließende Hydrolyse, wobei eine Ausgangsverbindung der Formel V oder X erhalten wird, wird vorzugsweise in einem wässrigen Lösungsmittel wie Ethanol/Wasser, Dioxan/Wasser oder Wasser und vorzugsweise in Gegenwart einer Säure wie Salzsäure oder Schwefelsäure oder in Gegenwart einer Base wie Natriumhydroxid oder Kaliumhydroxid bei erhöhten Temperaturen, vorzugsweise bei der Siedetemperatur des Reaktionsgemisches, durchgeführt.

Ausgehend von einer so erhaltenen Verbindung der Formel X erhält man besonders vorteilhaft gemäß dem vorstehend beschriebenen Verfahren c) eine Verbindung der Formel Ia wie dies im Anspruch 18

gekennzeichnet ist.

Wie bereits eingangs erwähnt, weisen die Verbindungen der allgemeinen Formel I wertvolle pharmakologische Eigenschaften auf, insbesondere antipyretische, analgetische und/oder antiphlogistische Eigenschaften.

Beispielsweise wurden die Verbindungen

A = 1-Methyl-7,8-dihydro-2,5(1H,6H)-chinolindion,
B = 3-Methyl-3,4,7,8-tetrahydro-2,5(1H,6H)-chinolindion,
C = 1-Methyl-3,4,7,8-tetrahydro-2,5(1H,6H)-chinolindion,
D = 4-Methyl-7,8-dihydro-2,5(1H,6H)-chinolindion,
E = 3-Methyl-7,8-dihydro-2,5(1H,6H)-chinolindion,
F = 1,3-Dimethyl-7,8-dihydro-2,5(1H,6H)-chinolindion,
G = 1-Ethyl-4-methyl-7,8-dihydro-2,5(1H,6H)-chinolindion,
H = 1-n-Butyl-4-methyl-7,8-dihydro-2,5(1H,6H)-chinolindion und
I = 1-Ethyl-3,4,7,8-tetrahydro-2,5(1H,6H)-chinolindion

wie folgt geprüft:

1. Die Prüfung der Wirkung gegen den Entzündungsschmerz der Ratte erfolgt mit der Methode nach RANDALL u. SELITTO (Arch. int. Pharmacodyn. 111, 409 (1957)). Die Prüfsubstanzen wurden männlichen Ratten mit einem Gewicht zwischen 100 und 130 g als Verreibung in 1%iger Methylcellulose (1,0 ml/100 g Tier) 90 bzw. 135 Minuten nach subcutaner Gabe der Hefe per Schlundsonde verabfolgt. Aus der 90 bzw. 45 Minuten nach Gabe der verschiedenen Dosen gemessenen Schmerzschwelle wurde nach linearer Regressionsanalyse eine $ED_{50}$ als jene Dosis ermittelt, die eine Anhebung der Schmerzschwelle um 50 % bewirkte.

Die nachfolgende Tabelle enthält die gefundenen Werte:

| Substanz | $ED_{50}$ mg/kg | |
|---|---|---|
| | nach 45 Minuten | nach 90 Minuten |
| A | 8,7 | 8,1 |
| B | 37,0 | 39,0 |
| C | 15,0 | 21,0 |
| D | 21,0 | 34,0 |
| E | 31,0 | 36,0 |
| F | 12,5 | 12,1 |
| G | 5,1 | 5,9 |
| H | | 19,1 |
| I | 13,0 | 16,0 |

2. Die Prüfung der Wirkung gegen den Wärmeschmerz der Maus erfolgte mit der Methode nach CHEN und BECKMAN (Science 113, 631 (1951)). Männliche Mäuse mit einem durchschnittlichen Gewicht von 20 g erhielten die Prüfsubstanzen als Verreibung in 1%iger Methylcellulose (0,1 ml/10 g Tier) per Schlundsonde. Aus der nach den verschiedenen Dosen beobachteten Verlängerung der individuellen Reaktionszeit wurde nach linearer Regressionsanalyse eine $Ed_{100}$ als jene Dosis berechnet, die zu einer Verdoppelung der Reaktionszeit führte.

Die nachfolgende Tabelle enthält die gefundenen Werte:

| Substanz | $ED_{100}$ mg/kg |
|---|---|
| A | 71 |
| B | 155 |
| C | 99 |
| D | 98 |
| E | 135 |
| F | 109 |
| G | 59 |
| H | 101 |
| I | 70 |

3. Die Prüfung der Wirkung gegen den mechanisch ausgelösten Schmerz erfolgte mit der Schwanzklemmenmethode nach HAFFNER (Dtsch. med. Wschr. 54, 731 (1929)). Männliche Mäuse mit einem Gewicht zwischen 19 und 24 g erhielten die Prüfsubstanzen als Verreibung in 1%iger Methylzellulose (0,1 ml/10 g Tier) per Schlundsonde. In Abständen von 30 Minuten wurde nach der Behandlung festgestellt, wieviel Mäuse nicht mehr auf das Anlegen der Klemme reagierten.

Aus dem Prozentsatz der Tiere, die nach den verschiedenen Dosen keine Schmerzreaktion zeigten, wurde mittels Probit-Analyse eine $ED_{50}$ berechnet.

Die nachfolgende Tabelle enthält die gefundenen Werte:

| Substanz | $ED_{50}$ mg/kg |
|---|---|
| A | 80 |
| B | 100 |
| C | 129 |
| D | 129 |
| E | 102 |
| F | 86 |
| G | 26 |
| H | 50 |
| I | 55 |

4. Die Prüfung der Wirkung auf die Körpertemperatur erfolgte an normothermen Ratten mit einem Gewicht zwischen 120 und 140 g. Die Prüfsubstanzen wurden als Verreibung in 1%iger Methylcellulose (1,0 ml/100 g Tier) per Schlundsonde verabfolgt. Aus der nach den verschiedenen Dosen beobachteten Senkung der Rektaltemperatur wurde nach linearer Regressionsanalyse eine $ED_{-1,5°C}$ als jene Dosis berechnet, die eine Senkung der Körpertemperatur um 1,5° C bewirkte.

Die nachfolgende Tabelle enthält die gefundenen Werte:

| Substanz | $ED_{-1,5°C}$ mg/kg |
|---|---|
| A | 29 |
| B | 35 |
| C | 26 |
| D | 25 |
| E | 34 |
| F | 36 |
| G | 11 |
| H | 36 |
| I | 22 |

5. Die Bestimmung der akuten Toxizität erfolgte an Mäusen oder Ratten beider Geschlechter mit einem mittleren Gewicht von 20 g. Die Prüfsubstanzen wurden als Verreibung in 1%iger Methylcellulose (0,2 ml/10

g Tier) per Schlundsonde verabfolgt. Die Berechnung der $LD_{50}$ erfolgte (soweit möglich) nach LITCHFIELD u. WILCOXON (J. Pharmacol. exp. Therap. 96, 99 (1949) aus dem Prozentsatz der Tiere, die innerhalb von 14 Tagen nach den verschiedenen Dosen verstarben.

Die nachfolgende Tabelle enthält die gefundenen Werte:

| Substanz | $LD_{50}$ mg/kg | |
|---|---|---|
| | Maus | Ratte |
| A | 767 | 489 |
| B | 1220 | 659 |
| C | 985 | 748 |
| D | - | 1370 |
| E | 825 | |
| F | 296 | 396 |
| G | - | - |
| H | - | - |
| I | - | - |

Aufgrund ihrer pharmakologischen Eigenschaften stellen die Verbindungen der allgemeinen Formel I ein Analgeticum/Antipyreticum vom Typ des Aminophenazons dar. Diese eignen sich daher zur Bekämpfung von Schmerzzuständen wie Kopf-, Zahn-, Regel-, Nervenschmerzen, Migräne, postoperativen und posttraumatischen Schmerzen, aber auch zur Bekämpfung von Fieber und Entzündungen bzw. der Symptomatik von Erkältungskrankheiten.

Hierzu lassen sich die Verbindungen der allgemeinen Formel I, gegebenenfalls in Kombination mit anderen Wirkstoffen, zusammen mit einem oder mehreren inerten Trägerstoffen und/oder Verdünnungsmitteln, z.B. mit Wasser, Maisstärke, Kartoffelstärke, Milchzucker, Rohrzucker, mikrokristalliner Cellulose, Magnesiumstearat, Polyvinylpyrrolidon, Hartfett, Carboxymethylcellulose oder deren geeignete Gemische, in übliche galenische Zubereitungen wie Tabletten, Dragées, Kapseln, Pulver, Zäpfchen, Suspensionen und Lösungen einarbeiten. Hierbei beträgt die Einzeldosis am Erwachsenen 25-1200 mg, zweckmäßigerweise 50-600 mg, vorzugsweise jedoch 100 bis 300 mg.

Die nachfolgenden Beispiele sollen die Erfindung näher erläutern:

## Beispiel A

### 7,8-Dihydro-2,5(1H,6H)-chinolindion

#### a) 7,8-Dihydro-2,5(1H,6H)-chinolindion-3-carbonsäure-ethylester

45,0 g (0,269 Mol) 2-Dimethylaminomethylen-cyclohexan-1,3-dion und 43 ml (1,5 x 0,269 Mol) Cyanessigsäure-ethylester werden in 400 ml absolutem Ethanol 1,5 Stunden zum Sieden am Rückfluß erhitzt. Nach dem Abkühlen werden die abgeschiedenen hellgelben Kristalle abgesaugt, mit wenig kaltem Ethanol gewaschen und getrocknet.

Schmelzpunkt: 227-233,5 °C,

Ausbeute: 60,8 g (96,1 % der Theorie).

#### b) 7,8-Dihydro-2,5(1H,6H)-chinolindion-3-carbonsäure

50,0 g 7,8-Dihydro-2,5(1H,6H)-chinolindion-3-carbonsäure-ethylester werden mit 1000 ml halbkonzentrierter Salzsäure 16 Stunden lang gerührt. Der gebildete kristalline Niederschlag wird abgesaugt, mit wenig Wasser gewaschen und bei 70 °C getrocknet.

Schmelzpunkt: 250 °C,

Ausbeute: 33,9 g (77,7 % der Theorie).

c) 7,8-Dihydro-2,5(1H,6H)-chinolindion

36,5 g (0,1762 Mol) 7,8-Dihydro-2,5(1H,6H)-chinolindion-3-carbonsäure werden zusammen mit 4,0 g Kupferpulver in 330 ml Chinolin unter Stickstoff in einem Ölbad von 230 °C erhitzt. Sobald die Innentemperatur 160 °C übersteigt, setzt eine zunächst langsame, dann lebhaftere Gasentwicklung ein, die nach 30 Minuten wieder abklingt und nach 40 Minuten beendet ist. Anschließend läßt man das Kupferpulver absitzen, dekantiert die überstehende, noch 160 °C heiße Lösung ab und rührt diese unter weiterer Abkühlung, zuletzt im Eisbad, wobei Kristallisation erfolgt. Das Rohprodukt wird abgesaugt und aus 400 ml Ethanol/Wasser (7:3) unter Zusatz von Aktivkohle umkristallisiert.
Schmelzpunkt: 302-303 °C,
Ausbeute: 16,6 g (57,6 % der Theorie).

Beispiel B

1-Methyl-7,8-dihydro-2,5(1H,6H)-chinolindion

3,36 g (0,0206 Mol) 7,8-Dihydro-2,5(1H,6H)-chinolindion werden zusammen mit 5,69 g (2 x 0,0206 Mol) gepulvertem wasserfreiem Kaliumcarbonat und 1,92 ml (1,5 x 0,0206 Mol) Methyljodid in 35 ml Dimethylformamid 16 Stunden lang bei Raumtemperatur gerührt. Anschließend saugt man ab, dampft das Lösemittel im Vakuum ab und kristallisiert aus Chloroform/Essigester um.
Schmelzpunkt: 203-205 °C,
Ausbeute: 3,1 g (84,9 % der Theorie).

Beispiel C

4-Methyl-7,8-dihydro-2,5(1H,6H)-chinolindion

a) 4-Methyl-5,6,7,8-tetrahydro-cumarin-5-on

231,2 g (2,0 Mol) Cyclohexan-1,3-dion (97%ig) werden in 670 ml absolutem Pyridin mit 325,3 g (1,25 x 2,0 Mol) Acetessigester und 4,88 g (0,02 x 2 Mol) 4-Dimethylaminopyridin unter Stickstoff 2,5 Stunden lang am Rückfluß zum Sieden erhitzt. Danach setzt man nochmals 65,1 g (0,25 x 2,0 Mol) Acetessigester zu und erhitzt weitere 3 Stunden zum Sieden. Danach wird das Pyridin im Vakuum abdestilliert und der ölige Rückstand noch warm mit 100 ml eines Gemisches aus Cyclohexan/Essigester (1:1) verrührt. Das auskristallisierte Reaktionsprodukt wird abgesaugt und getrocknet.
Ausbeute: 139,6 (39,2 % der Theorie)
Die Mutterlauge wird im Vakuum am Rotationsverdampfer vom Lösungsmittel befreit und der ölige Rückstand im Vakuum destilliert. Das Destillat erstarrt zu einer weißen kristallinen Substanz.
$Kp_{0,1-0,15}$ = 190-210 °C.
Ausbeute: 126 g (35,4 % der Theorie).
Gesamtausbeute: 265,6 g (74,5 % der Theorie).
Schmelzpunkt: 96-97 °C (nach Säulenchromatographie an Kieselgel mit Essigester/Methylenchlorid (1:1)).

Analog wurde folgende Verbindung hergestellt:
4-Ethyl-5,6,7,8-tetrahydro-cumarin-5-on
Schmelzpunkt: 54-55 °C

b) 4-Methyl-7,8-dihydro-2,5(1H,6H)-chinolindion

4,46 g (0,025 Mol) 4-Methyl-5,6,7,8-tetrahydro-cumarin-5-on werden im Rührautoklaven in 150 ml gesättigtem methanolischem Ammoniak 3 Stunden lang auf 180 °C erhitzt. Nach dem Abkühlen wird das methanolische Ammoniak am Rotationsverdampfer abdestilliert und der Rückstand aus Ethanol unter Verwendung von Aktivkohle umkristallisiert.

Schmelzpunkt: 278-280 °C,
Ausbeute: 4,01 g (64,7 % der Theorie).

Beispiel D

1,4-Dimethyl-7,8-dihydro-2,5(1H,6H)-chinolindion

4,46 g (0,025 Mol) 4-Methyl-5,6,7,8-tetrahydro-cumarin-5-on werden in einer Mischung von 41,9 g flüssigem Methylamin in 100 ml auf -9 °C vorgekühltem Methanol unter Kühlung gelöst und im Autoklaven 3 Stunden lang auf 180 °C erhitzt. Nach dem Abkühlen wird das Methanol und Methylamin am Rotationsverdampfer abdestilliert, der Rückstand an einer Kieselgelsäule mit Etyhlenchlorid/Ethanol (95:5) chromatographiert und aus Cyclohexan/Essigester (1:1) umkristallisiert.
Schmelzpunkt: 120-121,5 °C,
Ausbeute: 3,77 g (78,9 % der Theorie).

Beispiel E

3-Methyl-7,7-dimethyl-3,4,7,8-tetrahydro-2,5(1H,6H)-chinolindion

Hergestellt analog Beispiel G aus 1-Amino-5,5-dimethylcyclohexen-3-on und Methacrylsäure im Autoklaven bei 180 °C während einer Stunde.
Schmelzpunkt: 198-200 °C,
Ausbeute: 28,9 % der Theorie.

Beispiel F

4,7,7-Trimethyl-7,8-dihydro-2,5(1H,6H)-chinolindion

Hergestellt aus 4,7,7-Trimethyl-5,6,7,8-tetrahydro-cumarin-5-on und methanolischem Ammoniak analog Beispiel Cb.
Schmelzpunkt: 305-307 °C,
Ausbeute: 56,0 % der Theorie.

Beispiel G

3,4,7,8-Tetrahydro-2,5(1H,6H)-chinolindion

75,00 g (0,675 Mol) 1-Amino-cyclohexen-3-on und 65,65 g (1,35 x 0,675 Mol) Acrylsäure werden 3 Stunden lang unter Stickstoff in einem auf 180 °C erhitzten Ölbad gerührt. Es wird abgekühlt und aus 1000 ml Methanol umkristallisiert.
Schmelzpunkt: 192-194,5 °C,
Ausbeute: 45,1 % der Theorie.

Beispiel H

1-Methyl-3,4,7,8-tetrahydro-2,5(1H,6H)-chinolindion

Hergestellt aus 3,4,7,8-Tetrahydro-2,5(1H,6H)-chinolindion und Methyljodid mit Kaliumcarbonat in Aceton.
Schmelzpunkt: 72-74° C,
Ausbeute: 82,9 % der Theorie.

Beispiel I

1-Benzyl-3,4,7,8-tetrahydro-2,5(1H,6H)-chinolindion

Hergestellt analog Beispiel H aus 3,4,7,8-Tetrahydro-2,5(1H,6H)-chinolindion und Benzylbromid.
Schmelzpunkt: 63,5-65° C,
Ausbeute: 98,4 % der Theorie.

Beispiel K

3-Methyl-3,4,7,8-tetrahydro-2,5(1H,6H)-chinolindion

Hergestellt analog Beispiel G aus 1-Amino-cyclohexen-3-on und Methacrylsäure im Autoklaven während einer Stunde bei 180° C. Die Umkristallisation erfolgt aus Methanol und anschließend aus einem Gemisch von Essigester/Äthanol (3:1).
Schmelzpunkt: 210-212° C,
Ausbeute: 28,8 % der Theorie.

Beispiel L

7,7-Dimethyl-3,4,7,8-tetrahydro-2,5(1H,6H)-chinolindion

Hergestellt analog Beispiel G aus 1-Amino-5,5-dimethylcyclohexen-3-on und Acrylsäure bei 140° C während 3 Stunden.
Schmelzpunkt: 161-165,5° C,
Ausbeute: 95 % der Theorie.

Beispiel M

1-Methyl-7,8-dihydro-2,5(1H,6H)-chinolindion

a) 7,8-Dihydro-5-oxo(6H)cumarin-3-carbonsäureethylester

33,6 g (0,3 Mol) 97%iges 1,3-Cyclohexandion werden in 500 ml Dimethylformamid gelöst und unter Eiskühlung mit 33,7 g (0,3 Mol) Kalium-tert.-butylat versetzt. Anschließend gibt man 50,75 g (0,3 Mol) Ethoxymethylencyanessigsäureethylester, gelöst in 100 ml Dimethylformamid, innerhalb von 20 Minuten zu, wobei die Temperatur zwischen 15 und 20° C gehalten wird. Diese Lösung wird über Nacht bei Raumtemperatur gerührt, dann auf ein Gemisch von 750 g Eis und 320 ml 2N Salzsäure gegeben und 20 Minuten gerührt. Schließlich extrahiert man 5 mal mit 100 ml Essigester. Die organischen Phasen werden 5 mal mit gesättigter Natriumchloridlösung gewaschen und dann eingeengt.
Ausbeute: 65,9 g (93 % der Theorie)

Analog lassen sich folgende Verbindungen herstellen:
7,8-Dihydro-7-methyl-5-oxo(6H)cumarin-3-carbonsäureethylester
7,8-Dihydro-7,7-dimethyl-5-oxo(6H)cumarin-3-carbonsäureethylester
Schmelzpunkt: 93-95° C

b) 1-Methyl-7,8-dihydro-2,5(1H,6H)-chinolindion-3-carbonsäureethylester

40,58 g (0,17 Mol) 7,8-Dihydro-5-oxo-(5H)cumarin-3-carbonsäure-ethylester werden in 200 ml Ethanol gelöst und bei Raumtemperatur tropfenweise mit einem Äquivalent einer 5 molaren ethanolischen Methylaminolösung versetzt. Es fällt ein Niederschlag aus, der weitere 2 Stunden bei Raumtemperatur gerührt wird. Das Reaktionsgemisch wird auf 150 ml eingeengt und im Eisbad abgekühlt. Der Niederschlag wird abgesaugt und zweimal mit kaltem Ethanol gewaschen.
Schmelzpunkt: 140-142 °C,
Ausbeute: 35 g (81 % der Theorie).

c) 1-Methyl-7,8-dihydro-2,5(1H,6H)-chinolindion

29,1 g (0,5 Mol) fein pulverisierte Lithiumchlorid werden in 3,5 ml Wasser und 50 ml Dimethylsulfoxid suspendiert und mit 12,1 g (0,05 Mol) 1-Methyl-7,8-dihydro-2,5(1H,6H)-chinolindion-3-carbonsäure-ethylester portionsweise versetzt. Die Reaktionsmischung wird 6 Stunden in einem 190 °C heißen Ölbad gerührt. Nach Abklingen der Kohlendioxidentwicklung wird die Suspension auf 300 g Eis gegeben. Anschließend wird die Lösung mit 5 mal 100 ml Chloroform extrahiert. Die organischen Phasen werden mit 3 mal 50 ml gesättigter Natriumchloridlösung gewaschen, über Natriumsulfat getrocknet und einrotiert.
Schmelzpunkt: 205-208 °C (Ethanol),
Ausbeute: 5,9 g (67,3 % der Theorie).

Analog Beispiel M lassen sich folgende Verbindungen herstellen:
1-Ethyl-7,8-dihydro-2,5(1H,6H)-chinolindion
Schmelzpunkt: 149-150 °C
1-Methyl-7-methyl-7,8-dihydro-2,5(1H,6H)-chinolindion
1-Methyl-7,7-dimethyl-7,8-dihydro-2,5(1H,6H)-chinolindion
Schmelzpunkt: 130-131 °C

## Beispiel 1

1-Phenyl-7,8-dihydro-2,5(1H,6H)-chinolindion

a) 1-Phenyl-7,8-dihydro-2,5(1H,6H)-chinolindion-3-carbonsäure-ethylester

43 g (0,2 Mol) 2-Phenylaminomethylen-cyclohexan-1,3-dion werden bei 80 °C in 150 ml trockenem Dimethylformamid gelöst. Hierzu fügt man 29,2 g Cyanessigsäure-ethylester und tropft dann innerhalb 10 Minuten eine Lösung von 20 g (1,78 x 0,2 Mol) Kaliumhydroxid in 200 ml absolutem Äthanol zu. Die Reaktionsmischung erwärmt sich dabei bis zum beginnenden Sieden. Man rührt noch 5 Minuten bei 80 °C nach, kühlt auf 15 °C ab, gießt in 1000 ml Wasser und stellt mit konzentrierter Salzsäure auf pH 1,0. Nach Stehen über Nacht erhält man rosafarbene Kristalle, die man absaugt und trocknet.
Schmelzpunkt: 254 °C (Zers.),
Ausbeute: 36,9 g (659,3 % der Theorie).

b) 1-Phenyl-7,8-dihydro-2,5(1H,6H)-chinolindion-3-carbonsäure

61,7 g (0,2 Mol) 1-Phenyl-7,8-dihydro-2,5(1H,6H)-chinolindion-3-carbonsäure-ethylester werden in 750 ml 6n Salzsäure 48 Stunden lang bei Raumtemperatur gerührt. Man saugt das weiße Kristallisat von der leicht fluoreszierenden Lösung ab und trocknet.
Schmelzpunkt: > 270 °C,
Ausbeute: 52,2 g (92,1 % der Theorie).

c) 1-Phenyl-7,8-dihydro-2,5(1H,6H)-chinolindion

33,0 g (0,12 Mol) 1-Phenyl-7,8-dihydro-2,5(1H,6H)-chinolindion-3-carbonsäure werden analog Beispiel Ac in Chinolin unter Zusatz einer Spur Kupferpulver bei 140 °C decarboxyliert. Das Reaktionsgemisch wird mit 800 ml Petroläther versetzt und das ausgefällte graubraune Reaktionsprodukt abgesaugt. Man chromatographiert an einer Kieselgelsäule mit Essigester als Laufmittel.
Schmelzpunkt: 147-149,2 °C,
Ausbeute: 21,3 g (76,6 % der Theorie).

Beispiel 2

1-(4-Chlorphenyl)-7,8-dihydro-2,5(1H,6H)-chinolindion

Hergestellt analog Beispiel 1 ausgehend von 2-(4-Chlorphenyl)-aminomethylen-cyclohexan-1,3-dion, Verseifung und Decarboxylierung des so erhaltenen 1-(4-Chlorphenyl)-7,8-dihydro-2,5(1H,6H)-chinolindion-3-carbonsäure-ethylesters.
Schmelzpunkt: 196-199,5 °C,
Ausbeute: 60,4 % der Theorie.

Beispiel 3

1-(4-Methyl-phenyl)-7,8-dihydro-2,5(1H,6H)-chinolindion

Hergestellt analog Beispiel 1 ausgehend von 2-(4-Methyl-phenyl)-aminomethylen-cyclohexan-1,3-dion und anschließender Verseifung und Decarboxylierung des so erhaltenen 1-(4-Methyl-phenyl)-7,8-dihydro-2,5(1H,6H)-chinolindion-3-carbonsäure-ethylesters.
Schmelzpunkt: 192-194 °C,
Ausbeute: 53,3 % der Theorie.

Beispiel 4

1-(4-Methoxyphenyl)-7,8-dihydro-2,5(1H,6H)-chinolindion

Hergestellt analog Beispiel 1 ausgehend von 2-(4-Methoxyphenyl)-aminomethylen-cyclohexan-1,3-dion und anschließende Verseifung und Decarboxylierung des so erhaltenen 1-(4-Methoxy-phenyl)-7,8-dihydro-2,5(1H,6H)-chinolindion-3-carbonsäure-ethylesters.
Schmelzpunkt: 164-166 °C,
Ausbeute: 28,6 % der Theorie.

Beispiel 5

3-Methyl-7,8-dihydro-2,5(1H,6H)-chinolindion

a) 3-Methyl-5,6,7,8-tetrahydro-cumarin-5-on

Hergestellt analog Beispiel Ca aus Cyclohexan-1,3-dion und 2-Formyl-propionsäure-ethylester.
Schmelzpunkt: 83-86 °C,
Ausbeute: 75,7 % der Theorie.

b) 3-Methyl-7,8-dihydro-2,5(1H,6H)-chinolindion

3,0 g (0,0168 Mol) 3-Methyl-5,6,7,8-tetrahydro-cumarin-5-on werden in 50 ml gesättigtem methanolischem Ammoniak gelöst und 2 Tage bei Raumtemperatur stehen gelassen. Danach werden die ausgeschiedenen, glänzenden, plättchenförmigen Kristalle abgesaugt, mit wenig kaltem Methanol gewaschen und getrocknet.
Schmelzpunkt: 287-290 °C (Zers.),
Ausbeute: 2,16 g (72,6 % der Theorie).

Beispiel 6

1,3-Dimethyl-7,8-dihydro-2,5(1H,6H)-chinolindion

Hergestellt analog Beispiel 5b) aus 3-Methyl-5,6,7,8-tetrahydro-cumarin-5-on und methanolischer Methylaminolösung bei Zimmertemperatur. Nach Abdampfen des methanolischen Ammoniaks wird aus Wasser unter Zusatz von Kohle umkristallisiert.
Schmelzpunkt: 127-129 °C,
Ausbeute: 70,2 % der Theorie.

Beispiel 7

1-Ethyl-3-methyl-7,8-dihydro-2,5(1H,6H)-chinolindion

Hergestellt analog Beispiel 5b) aus 3-Methyl-5,6,7,8-tetrahydro-cumarin-5-on und Ethylamin.
Schmelzpunkt: 155-160 °C,
Ausbeute: 81,9 % der Theorie.

Beispiel 8

1-(2-Hydroxyethyl)-3-methyl-7,8-dihydro-2,5(1H,6H)-chinolindion

Hergestellt analog Beispiel 5b) aus 3-Methyl-5,6,7,8-tetrahydro-cumarin-5-on und 2-Hydroxy-ethylamin.
Schmelzpunkt: 146-152 °C,
Ausbeute: 88,4 % der Theorie.

Beispiel 9

1-(2-Methylmercapto-ethyl)-3-methyl-7,8-dihydro-2,5(1H,6H)-chinolindion

Hergestellt analog Beispiel 5b) aus 3-Methyl-5,6,7,8-tetrahydro-cumarin-5-on und 2-Methylmercapto-ethylamin.
Schmelzpunkt: 86-88,5 °C,
Ausbeute: 53,1 % der Theorie.

Beispiel 10

1-(2-Hydroxy-ethyl)-4-methyl-7,8-dihydro-2,5(1H,6H)-chinolindion

Hergestellt analog Beispiel 5b) aus 4-Methyl-5,6,7,8-tetrahydro-cumarin-5-on und 2-Hydroxy-ethylamin.
Schmelzpunkt: 111-112° C,
Ausbeute: 91,6 % der Theorie.

Beispiel 11

1-(2-Methoxy-ethyl)-4-methyl-7,8-dihydro-2,5(1H,6H)-chinolindion

Hergestellt analog Beispiel 5b) aus 4-Methyl-5,6,7,8-tetrahydro-cumarin-5-on und 2-Methoxy-ethylamin.
Schmelzpunkt: 105-107° C,
Ausbeute: 83,7 % der Theorie.

Beispiel 12

1-Isopropyl-4-methyl-7,8-dihydro-2,5(1H,6H)-chinolindion

Hergestellt analog Beispiel 5b) aus 4-Methyl-5,6,7,8-tetrahydro-cumarin-5-on und Isopropylamin.
Schmelzpunkt: 89,5-90,5° C,
Ausbeute: 16,8 % der Theorie.

Beispiel 13

1-n-Butyl-4-methyl-7,8-dihydro-2,5(1H,6H)-chinolindion

Hergestellt analog Beispiel 5b) aus 4-Methyl-5,6,7,8-tetrahydro-cumarin-5-on und n-Butylamin.
Schmelzpunkt: 58,5-60° C,
Ausbeute: 86,1 % der Theorie.

Beispiel 14

1-Methyl-4-trifluormethyl-7,8-dihydro-2,5(1H,6H)-chinolindion

a) 4-Trifluormethyl-5,6,7,8-tetrahydro-cumarin-5-on

Hergestellt analog Beispiel Ca aus Cyclohexan-1,3-dion und ω,ω,ω-Trifluoracetessigsäure-ethylester.
Schmelzpunkt: 78-80° C,
Ausbeute: 10,1 % der Theorie.

b) 1-Methyl-4-trifluormethyl-7,8-dihydro-2,5(1H,6H)-chinolindion

Hergestellt analog Beispiel 5b) aus 4-Trifluormethyl-5,6,7,8-tetrahydro-cumarin-5-on und Methylamin bei Raumtemperatur.
Schmelzpunkt: 98-99° C,
Ausbeute: 8 % der Theorie.

Beispiel 15

4-Ethyl-7,8-dihydro-2,5(1H,6H)-chinolindion

Hergestellt aus 4-Ethyl-5,6,7,8-tetrahydro-cumarin-5-on (hergestellt aus 3-Oxo-n-valeriansäure-ethylester und Cyclohexan-1,3-dion) und methanolischem Ammoniak im Autoklaven während einer Stunde bei 140 °C.
Schmelzpunkt: 218-220 °C,
Ausbeute: 62,0 % der Theorie.

Beispiel 16

1-Methyl-4-ethyl-7,8-dihydro-2,5(1H,6H)-chinolindion

Hergestellt aus 4-Ethyl-5,6,7,8-tetrahydro-cumarin-5-on und methanolischer Methylaminlösung während 31 Stunden bei Raumtemperatur.
Schmelzpunkt: 102-103 °C,
Ausbeute: 78 % der Theorie.

Beispiel 17

3,4-Dimethyl-7,8-dihydro-2,5(1H,6H)-chinolindion

Hergestellt aus 3,4-Dimethyl-5,6,7,8-tetrahydro-cumarin-5-on (Schmelzpunkt: 109-111 °C, hergestellt aus 2-Methylacetessigmethylester und Cyclohexan-1,3-dion) und methanolischem Ammoniak während einer Stunde im Autoklaven bei 150 °C.
Schmelzpunkt: 274-276 °C,
Ausbeute: 72,0 % der Theorie.

Beispiel 18

1,3,4-Trimethyl-7,8-dihydro-2,5(1H,6H)-chinolindion

Hergestellt aus 3,4-Dimethyl-5,6,7,8-tetrahydro-cumarin-5-on und methanolischer Methylaminlösung während 15 Stunden bei Raumtemperatur.
Schmelzpunkt: 197-199 °C.,
Ausbeute: 98,6 % der Theorie.

Beispiel 19

1-(2-Hydroxy-ethyl)-3,4,-dimethyl-7,8-dihydro-2,5(1H,6H)-chinolindion

Hergestellt aus 3,4-Dimethyl-5,6,7,8-tetrahydro-cumarin-5-on und 2-Hydroxy-ethylamin bei 130 °C während 25 Minuten.
Schmelzpunkt: 140-141 °C,
Ausbeute: 56,0 % der Theorie.

Beispiel 20

2,3,6,7-Tetrahydro-4,9(1H,5H,8H)-cyclopenta[c]chinolindion

Hergestellt aus 2,3,6,7-Tetrahydro-4,9(1H,8H)-cyclopenta[c][1]benzopyrandion (hergestellt aus Cyclohexan-1,3-dion und Cyclopentanon-2-carbonsäure-ethylester) mit methanolischem Ammoniak während einer Stunde bei 140° C im Autoklaven.
Schmelzpunkt: 305-312° C,
Ausbeute: 91,2 % der Theorie.

Beispiel 21

5-Methyl-2,3,6,7-tetrahydro-4,9(1H,5H,8H)-cyclopenta[c]chinolindion

Hergestellt aus 2,3,6,7-Tetrahydro-4,9(1H,8H)-cyclopenta[c][1]benzopyrandion und Methylamin bei Raumtemperatur während 1 1/2 Stunden.
Schmelzpunkt: 224-226° C,
Ausbeute: 92,1 % der Theorie.

Beispiel 22

5-(2-Hydroxy-ethyl)-2,3,6,7-tetrahydro-4,9(1H,5H,8H)-cyclopenta[c]chinolindion

Hergestellt aus 2,3,6,7-Tetrahydro-4,9(1H,8H)-cyclopenta[c][1]benzopyrandion und 2-Hydroxy-ethylamin.
Schmelzpunkt: 162,5-163,5° C,
Ausbeute: 81,1 % der Theorie.

Beispiel 23

3,4,7,8,9,10-Hexahydro-1,6(2H,5H)-phenanthridindion

Hergestellt aus 3,4,7,8,9,10-Hexahydro-1,6(2H)-dibenzo[b,d]pyran (hergestellt aus Cyclohexan-1,3-dion und Cyclohexanon-2-carbonsäure-ethylester) mit methanolischem Ammoniak während einer Stunde im Autoklaven bei 140° C.
Schmelzpunkt: 303-306° C,
Ausbeute: 91,7 % der Theorie.

Beispiel 24

5-(2-Hydroxy-ethyl)-3,4,7,8,9,10-hexahydro-1,6(2H,5H)-phenanthridindion

Hergestellt aus 3,4,7,8,9,10-Hexahydro-1,6(2H)-dibenzo[b,d]pyran und 2-Hydroxy-ethylamin bei 125° C während 55 Minuten.
Schmelzpunkt: 152-153° C,
Ausbeute: 77,1 % der Theorie.

Beispiel 25

4-Phenyl-7,8-dihydro-2,5(1H,6H)-chinolindion

Hergestellt aus 4-Phenyl-5,6,7,8-tetrahydro-cumarin-5-on (Schmelzpunkt: 167-168° C, hergestellt aus

Benzoylessigsäureethylester und Cyclohexan-1,3-dion) und methanolischem Ammoniak im Autoklaven während einer Stunde bei 135° C.
Schmelzpunkt: 278-281° C,
Ausbeute: 82,5 % der Theorie.

Beispiel 26

1-Methyl-4-phenyl-7,8-dihydro-2,5(1H,6H)-chinolindion

Hergestellt aus 4-Phenyl-5,6,7,8-tetrahydro-cumarin-5-on und methanolischem Methylamin bei Raumtemperatur.
Schmelzpunkt: 164-165° C,
Ausbeute: 85,7 % der Theorie.

Beispiel 27

1-Ethyl-3,4,7,8-tetrahydro-2,5(1H,6H)-chinolindion

21,2 g (0,1 Mol) 2-(2-Carbäthoxy-ethyl)-cyclohexan-1,3-dion und 200 ml 24%ige äthanolische Ethylaminlösung werden im Rührautoklaven 3 Stunden auf 180° C erhitzt. Die Reaktionslössung wird am Rotationsverdampfer eingedampft und der Rückstand an einer Kieselgelsäule mit Cyclohexan/Essigsäure-ethylester (1:1) chromatographiert.
Schmelzpunkt: 88-92° C,
Ausbeute: 11,6 g (60,0 % der Theorie).

Beispiel 28

1-(2-Hydroxyethyl)-3,4,7,8-tetrahydro-2,5(1H,6H)-chinolindion

Hergestellt analog Beispiel 27 aus 2-(2-Carbäthoxyethyl)-cyclohexan-1,3-dion und 2-Hydroxy-ethylamin.
Schmelzpunkt: 103-104,5° C,
Ausbeute: 45 % der Theorie.

Beispiel 29

1-Carbomethoxymethyl-3,4,7,8-tetrahydro-2,5(1H,6H)-chinolindion

3,17 g (0,019 Mol) 3,4,7,8-Tetrahydro-2,5(1H,6H)-chinolindion werden in 25 ml Dimethylformamid gelöst, mit 5,3 g (1,2 x 0,019 Mol) wasserfreiem Kaliumcarbonat und anschließend mit 3,52 g (2 x 0,019 Mol) Bromessigsäuremethylester versetzt und 16 Stunden lang bei Zimmertemperatur gerührt. Man neutralisiert mit methanolischer Salzsäure und dampft am Rotationsverdampfer ein. Der Rückstand wird durch Säulenchromatographie gereinigt (Kieselgel, Laufmittel: Cyclohexan/Essigester = 1:1).
Schmelzpunkt: 87-89° C,
Ausbeute: 3,46 g (76 % der Theorie).

Beispiel 30

1-(4-Chlorbenzyl)-3,4,7,8-tetrahydro-2,5(1H,6H)-chinolindion

Hergestellt analog Beispiel 29 aus 3,4,7,8-Tetrahydro-2,5(1H,6H)-chinolindion und 4-Chlorbenzylchlorid.
Schmelzpunkt: 100-102° C,
Ausbeute: 55,4 % der Theorie.

Beispiel 31

1-(2-Phenylethyl)-3,4,7,8-tetrahydro-2,5(1H,6H)-chinolindion

Hergestellt analog Beispiel 27 aus 2-(2-Carbäthoxy-ethyl)-cyclohexan-1,3-dion und 2-Phenylethylamin in siedendem Xylol am Wasserabscheider.
Schmelzpunkt: 94,5-97° C,
Ausbeute: 36,8 % der Theorie.

Beispiel 32

1,3-Dimethyl-3,4,7,8-tetrahydro-2,5(1H,6H)-chinolindion

Hergestellt analog Beispiel 29 aus 3-Methyl-3,4,7,8-tetrahydro-2,5(1H,6H)-chinolindion und Methyljodid in siedendem Aceton.
Schmelzpunkt: 63-64° C,
Ausbeute: 84,5 % der Theorie.

Beispiel 33

1-Äthyl-4-methyl-7,8-dihydro-2,5(1H,6H)-chinolindion

12,5 g (0,07 Mol) 4-Methyl-5,6,7,8-tetrahydro-cumarin-5-on werden in einer 24%igen ethanolischen Ethylaminlösung gelöst und 3 Tage bei Raumtemperatur stehen gelassen. Danach wird abgedampft und der Rückstand aus Essigester/Cyclohexan umkristallisiert.
Schmelzpunkt: 97-98° C,
Ausbeute: 13,3 g (92,6 % der Theorie).

Analog Beispiel 33 wurden folgende Verbindungen hergestellt:
1-Propyl-4-methyl-7,8-dihydro-2,5(1H,6H)-chinolindion
Schmelzpunkt: 108-109° C
1-Isopropyl-4-methyl-7,8-dihydro-2,5(1H,6H)-chinolindion
Schmelzpunkt: 89,5-90,5° C
4-Ethyl-1-methyl-7,8-dihydro-2,5(1H,6H)-chinolindion
Schmelzpunkt: 102-103° C
1,4-Diethyl-7,8-dihydro-2,5(1H,6H)-chinolindion
Schmelzpunkt: 65-70° C

Beispiel 34

1-(2-Methoxy-ethyl)-7,8-dihydro-2,5(1H,6H)-chinolindion

a) 1-(2-Methoxy-ethyl)-7,8-dihydro-2,5(1H,6H)-chinolindion-3-carbonsäureethylester

23,6 g (0,1 Mol) 7,8-Dihydro-5-oxo-(5H)cumarin-3-carbonsäure-ethylester werden in 40 ml Ethanol gelöst und bei Raumtemperatur tropfenweise mit 7,5 g (0,1 Mol) 2-Methoxy-ethylamin, gelöst in 20 ml

Ethanol, versetzt. Es fällt ein Niederschlag aus, der weitere 2 Stunden bei Raumtemperatur gerührt wird. Das Reaktionsgemisch wird eingeengt, der Niederschlag abgesaugt, in 500 ml Essigsäure-ethylester gelöst und über eine Kieselgelsäule filtriert.
Ausbeute: 14,3 g Öl (48 % der Theorie).

b) 1-(2-Methoxy-methyl)-7,8-dihydro-2,5(1H,6H)-chinolindion

21,9 g (0,375 Mol) fein pulverisierte Natriumchlorid werden in 2,7 ml Wasser und 60 ml Dimethylsulfoxid suspendiert und mit 11,0 g (0,375 Mol) 1-(2-Methoxy-methyl)-7,8-dihydro-2,5(1H,6H)-chinolindion-3-carbonsäure-ethylester portionsweise versetzt. Die Reaktionsmischung wird 6 Stunden in einem 190 °C heißen Ölbad gerührt. Nach Abklingen der Kohlendioxidentwicklung wird die Suspension auf 300 g Eis gegeben. Anschließend wird die Lösung 3 mal mit 150 ml Chloroform extrahiert. Die organischen Phasen werden 5 mal mit 50 ml gesät tigter Natriumchloridlösung gewaschen, über Natriumsulfat getrocknet und einrotiert. Anschließend wird über eine Kieselgelsäule chromatographiert (Laufmittel: Essigsäureethylester/Methanol = 15/1).
Schmelzpunkt: 119-121 °C,
Ausbeute: 3,0 g (36 % der Theorie).

Analog Beispiel 34 wurden folgende Verbindungen hergestellt:
1-(2-Hydroxy-ethyl)-7,8-dihydro-2,5(1H,6H)-chinolindion
Schmelzpunkt: 145-148 °C
1-(3-Methoxy-propyl)-7,8-dihydro-2,5(1H,6H)-chinolindion
Schmelzpunkt: 98-102 °C
1-Propyl-7,8-dihydro-2,5(1H,6H)-chinolindion
Schmelzpunkt: 69-70 °C
1-Isopropyl-7,8-dihydro-2,5(1H,6H)-chinolindion
Schmelzpunkt: 110-112 °C
1-Butyl-7,8-dihydro-2,5(1H,6H)-chinolindion
Schmelzpunkt: 64-70 °C
1-(2-Methylpropyl)-7,8-dihydro-2,5(1H,6H)-chinolindion
Öl, Rf-Wert: 0,65 (Polygram-Kieselgelplatten SIL G/UV 254 der Firma Macherey-Nagel, Laufmittel: Chloroform/Ethanol = 19/1)
1-Tetrahydrofurforyl-7,8-dihydro-2,5(1H,6H)-chinolindion
Öl, Rf-Wert: 0,58 (Polygram-Kieselgelplatten SIL G/UV 254 der Firma Macherey-Nagel, Laufmittel: Chloroform/Ethanol = 19/1)
1-Cyclohexyl-7,8-dihydro-2,5(1H,6H)-chinolindion
Schmelzpunkt: 162-163 °C
1-Allyl-7,8-dihydro-2,5(1H,6H)-chinolindion
Schmelzpunkt: 115-117 °C
1-Propargyl-7,8-dihydro-2,5(1H,6H)-chinolindion
Schmelzpunkt: 208-210 °C

## Beispiel 35

1-Methoxycarbonylmethyl-7,8-dihydro-2,5(1H,6H)-chinolindion

Hergestellt aus 7,8-Dihydro-2,5(1H,6H)-chinolindion und Bromessigsäure-methylester analog Beispiel 29.
Schmelzpunkt: 129-130 °C

## Beispiel 36

1-Carboxylmethyl-7,8-dihydro-2,5(1H,6H)-chinolindion

Hergestellt durch Verseifung von 1-Methoxycarbonylmethyl-7,8-dihydro-2,5(1H,6H)-chinolindion in 1N-Natronlauge bei Raumtemperatur. Nach 2 Stunden wird auf pH 2 eingestellt und mit Essigsäure-ethylester extrahiert.
Schmelzpunkt: 238-240 °C (Zers.)

Beispiel I

Tabletten mit 125 mg 1-Methyl-7,8-dihydro-2,5(1H,6H)-chinolindion

Zusammensetzung:

| | |
|---|---|
| Wirkstoff | 125,0 mg |
| mikrokristalline Cellulose | 63,5 mg |
| Milchzucker, direkttablettierbar | 110,0 mg |
| Magnesiumstearat | 1,5 mg |
| | 300,0 mg |

Herstellung:

Die Hilfsstoffe werden mit dem Wirkstoff gründlich gemischt und zu Tabletten verpreßt. Man erhält runde, biplane Tabletten mit beidseitiger Facette und einseitiger Teilkerbe.
Tabletten-Gewicht: ca. 300 mg
Tabletten-Durchmesser: 10 mm

Beispiel II

Dragees mit 125 mg 1-Methyl-7,8-dihydro-2,5(1H,6H)-chinolindion

Zusammensetzung:

| | |
|---|---|
| Wirkstoff | 125,0 mg |
| mikrokristalline Cellulose | 63,5 mg |
| Milchzucker, direkttablettierbar | 110,0 mg |
| Magnesiumstearat | 1,5 mg |
| | 300,0 mg |

Herstellung:

Die Hilfsstoffe werden mit dem Wirkstoff gründlich gemischt und zu Dragees verpreßt. Die erhaltenen Dragees werden anschließend mit üblicher Zuckerdragiersuspension und anschließend mit reinem Zuckersirup auf 390 mg/Dragee in einem Dragierkessel dragiert.
Drageekern-Gewicht: ca. 300 mg
Drageekern-Durchmesser: 10 mm
Aussehen: rund, bikonvex

Beispiel III

Tabletten mit 125 mg 1-Ethyl-3,4,7,8-tetrahydro-2,5(1H,6H)-chinolindion

Zusammensetzung:

| | |
|---|---|
| Wirkstoff | 125,0 mg |
| Milchzucker | 75,0 mg |
| Maisstärke | 33,8 mg |
| Polyvinylpyrrolidon (Kollidon 25®) | 5,0 mg |
| Magnesiumstearat | 1,2 mg |
| | 240,0 mg |

Herstellung:

Der mit Milchzucker und Maisstärke gründlich gemischte Wirkstoff wird mit der alkoholischen Polyvinylpyrrolidonlösung gleichmäßig durchfeuchtet, die Masse durch 2,5 mm Maschenweite gesiebt, getrocknet und durch 1,5 mm Maschenweite erneut gesiebt. Dem so erhaltenen Granulat wird das Schmiermittel zugemischt (= preßfertige Mischung). Die Mischung wird zu Tabletten verpreßt. Man erhält runde, biplane Tabletten mit beidseitiger Facette und einseitiger Teilkerbe.
Tabletten-Gewicht: ca. 240 mg
Tabletten-Durchmesser: 9 mm

Beispiel IV

Dragees mit 125 mg 1-Ethyl-3,4,7,8-tetrahydro-2,5(1H,6H)-chinolindion

Zusammensetzung:

| | |
|---|---|
| Wirkstoff | 125,0 mg |
| Milchzucker | 75,0 mg |
| Maisstärke | 33,8 mg |
| Polyvinylpyrrolidon (Kollidon 25®) | 5,0 mg |
| Magnesiumstearat | 1,2 mg |
| | 240,0 mg |

Herstellung:

Der mit Milchzucker und Maisstärke gründlich gemischte Wirkstoff wird mit der alkoholischen Polyvinylpyrrolidonlösung gleichmäßig durchfeuchtet, die Masse durch 2,5 mm Maschenweite gesiebt, getrocknet und durch 1,5 mm Maschenweite erneut gesiebt. Die Mischung wird zu Drageekernen verpreßt. Die erhaltenen Dragees werden anschließend mit üblicher Zuckerdragiersuspension und anschließend mit reinem Zuckersirup auf 290 mg/Dragee in einem Dragierkessel dragiert.
Drageekern-Gewicht: ca. 240 mg
Drageekern-Durchmesser: 9 mm
Aussehen: rund, bikonvex

Beispiel V

Granulat mit 1-Methyl-7,8-dihydro-2,5(1H,6H)-chinolindion

Zusammensetzung:

| | |
|---|---|
| (01) Wirkstoff | 12,5 % |
| (02) Sorbit | 86,0 % |
| (03) Siliciumdioxid | 1,3 % |
| (04) Magnesiumstearat | 0,2 % |
| | 100,0 % |

Herstellung:

Die Komponenten (01), (02) und (03) werden gemischt und mit Äthanol feucht granuliert. Nach Trocknung und Sieben (Maschenweite 1,0 mm) wird das Granulat mit (04) gemischt und in Sachets abgefüllt. Ein Sachet mit 1 g Granulat enthält 125 mg Wirkstoff.

Beispiel VI

"Fine Granules" mit 1-Ethyl-3,4,7,8-tetrahydro-2,5(1H,6H)-chinolindion

Zusammensetzung:

| | |
|---|---|
| (01) Wirkstoff | 12,5 % |
| (02) Polyvinylpyrrolidon | 2,8 % |
| (03) Lactose | 83,0 % |
| (04) Siliciumdioxid | 1,2 % |
| (05) Magnesiumstearat | 0,5 % |
| | 100,0 % |

Herstellung:

In einem rotierenden Dragierkessel wird (03) mit einer Teilchengröße von 0,2-0,45 mm vorgelegt.
In Isopropanol wird (02) gelöst und anschließend (01) und (04) suspendiert. Die Suspension wird unter Zuführung von Trockenluft vorsichtig auf die im Kessel fließenden Lactose-Kristalle aufgesprüht. Nach Trocknung mischt man mit (05) und füllt eine 125 mg Wirkstoff entsprechende Menge Granules in Sachets ab.

Beispiel VII

Hartgelatinekapseln mit 125 mg 1-Ethyl-3,4,7,8-tetrahydro-2,5(1H,6H)-chinolindion

Zusammensetzung:

| 1. Kapselfüllgewicht: | |
|---|---|
| Wirkstoff | 125,0 mg |
| Lactose x $H_2O$ | 41,0 mg |
| Maisstärke getr. | 82,0 mg |
| Magnesiumstearat | 2,0 mg |
| | 250,0 mg |

| 2. Granulierflüssigkeit: | |
|---|---|
| Wasser, dest.<br>Hartgelatinekapsel Größe 2 | q.s. |

Herstellung:

Granulierflüssigkeit:

Ein Teil der Lactose wird ca. 20%ig in erwärmtem dest. Wasser gelöst.

Granulat:

Der Wirstoff, die restliche Lactose und Maisstärke werden gemischt und mit der Granulierflüssigkeit durchfeuchtet. Die Masse wird gesiebt, getrocknet und nach nochmaligem Sieben mit Magnesiumstearat homogen gemischt.
Die feinkörnige Endmischung wird auf einer geeigneten Maschine in Hartgelatinekapseln Größe 2 abgefüllt.

Beispiel VIII

Injektionslösung mit 125 mg 1-Ethyl-3,4,7,8-tetrahydro-2,5(1H,6H)-chinolindion

Zusammensetzung:

| | |
|---|---|
| Wirkstoff | 125,0 mg |
| Wasser für Injektionswecke ad | 2 ml |

Herstellung:

Der Wirkstoff wird bei Raumtemperatur in Wasser für Injektionszwecke gelöst. Die Lösung wird sterilfiltriert, in gereinigte Ampullen abgefüllt und bei 121 °C 20 Minuten lang autoklaviert.

Beispiel IX

Injektionslösung mit 125 mg 1-Ethyl-3,4,7,8-tetrahydro-2,5(1H,6H)-chinolindion

Zusammensetzung:

| | |
|---|---|
| Wirkstoff | 125,0 mg |
| Polyethylenglykol | 200,0 mg |
| Polyoxyethylen/Polyoxypropylen | 100,0 mg |
| Wasser für Injektionszwecke ad | 2 ml |

Herstellung:

Der Wirkstoff wird bei Raumtemperatur in Wasser für Injektionszwecke, in Polyethylenglykol mit einem durchschnittlichen Molgewicht von 600 und in Polyoxyethylen/Polyoxypropylen-Polymerisat gelöst. Die Lösung wird sterilfiltriert, in gereinigte Ampullen abgefüllt und bei 121 °C 20 Minuten lang autoklaviert.

Beispiel X

Injektionslösung mit 125 mg 1-Methyl-7,8-dihydro-2,5(1H,6H)-chinolindion

Zusammensetzung:

| | |
|---|---|
| Wirkstoff | 125,0 mg |
| Polyethylenglykol | 200,0 mg |
| Wasser für Injektionszwecke ad | 2 ml |

Herstellung:

Siehe Beispiel IX.

Beispiel XI

Suppositorien mit 200 mg 1-Methyl-7,8-dihydro-2,5(1H,6H)-chinolindion

Zusammensetzung:

| | |
|---|---|
| Wirkstoff | 200,0 mg |
| Hartfett (z.B. Witepsol H 19 und Witepsol W 45) | 1.500,0 mg |

Herstellung:

Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen ausgegossen.

Beispiel XII

Suppositorien mit 200 mg 1-Ethyl-3,4,7,8-tetrahydro-2,5(1H,6H)-chinolindion

Zusammensetzung:

| | |
|---|---|
| Wirkstoff | 200,0 mg |
| Hartfett (z.B. Witepsol H 19 und Witepsol W 45) | 1.500,0 mg |

Herstellung:

Siehe Beispiel XI.

Beispiel XIII

Saft mit 125 mg 1-Ethyl-3,4,7,8-tetrahydro-2,5(1H,6H)-chinolindion pro 5 ml

Zusammensetzung:

| | | |
|---|---|---|
| Wirkstoff | | 2,50 g |
| Carboxymethylcellulose | | 0,10 g |
| p-Hydroxybenzoesäuremethylester | | 0,05 g |
| p-Hydroxybenzoesäurepropylester | | 0,03 g |
| Saccharose | | 10,00 g |
| Glycerin | | 5,00 g |
| Sorbitlösung 70 % | | 20,00 g |
| Aroma | | 0,30 g |
| Wasser dest. | ad | 100,00 ml |

Herstellung:

Destilliertes Wasser wird auf 70 °C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und gelöst. Nach Zugabe und Lösen der Saccharose, der Sorbitlösung und des Aromas wird der Saft zur Entlüftung unter Rühren evakuiert.

Beispiel XIV

Saft mit 125 mg 1-Methyl-7,8-dihydro-2,5(1H,6H)-chinolindion pro 5 ml

Zusammensetzung:

Wirkstoff 2,50 g

| | | |
|---|---|---|
| Carboxymethylcellulose | | 0,10 g |
| p-Hydroxybenzoesäuremethylester | | 0,05 g |
| p-Hydroxybenzoesäurepropylester | | 0,03 g |
| Saccharose | | 10,00 g |
| Glycerin | | 5,00 g |
| Sorbitlösung 70 % | | 20,00 g |
| Aroma | | 0,30 g |
| Wasser dest. | ad | 100,00 ml |

Herstellung:

Siehe Beispiel XIII.

## Ansprüche

1. Arzneimittel, enthaltend eine Verbindung der Formel

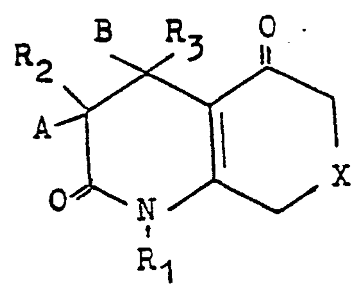

,(I)

in der
A und B jeweils ein Wasserstoffatom oder zusammen eine Kohlenstoff-Kohlenstoff-Bindung,
$R_1$ ein Wasserstoffatom, eine gegebenenfalls durch eine Phenyl-, Fluorphenyl-, Chlorphenyl- oder Bromphenylgruppe, durch eine Carboxygruppe oder durch eine Alkoxycarbonylgruppe mit insgesamt 2 bis 4 Kohlenstoffatomen substituierte Alkylgruppe mit 1 bis 3 Kohlenstoffatomen, eine Alkylgruppe mit 4 bis 6 Kohlenstoffatomen, eine Cycloalkylgruppe mit 3 bis 6 Kohlenstoffatomen, eine Alkenyl- oder Alkinylgruppe mit jeweils 3 bis 5 Kohlenstoffatomen, eine in 2- oder 3-Stellung durch eine Hydroxy-, Alkoxy- oder Alkylmercaptogruppe substituierte Alkylgruppe mit 2 oder 3 Kohlenstoffatomen, in welcher der Alkoxy- oder Alkylmercaptosubstituent jeweils 1 bis 3 Kohlenstoffatome enthalten kann, eine gegebenenfalls durch ein Halogenatom, durch eine Alkyl- oder Alkoxygruppe mit jeweils 1 bis 3 Kohlenstoffatome im Alkylteil substituierte Phenylgruppe oder eine Tetrahydrofurforylgruppe,
$R_2$ ein Wasserstoffatom oder eine Alkylgruppe mit 1 bis 3 Kohlenstoffatomen,
$R_3$ ein Wasserstoffatom, eine Trifluormethyl-, Phenyl- oder Alkylgruppe mit 1 bis 3 Kohlenstoffatomen oder
$R_2$ und $R_3$ zusammen eine n-Alkylengruppe mit 3 bis 5 Kohlenstoffatomen und
X eine gegebenenfalls durch eine oder zwei Alkylgruppen mit jeweils 1 bis 3 Kohlenstoffatomen substituierte Methylengruppe bedeuten, neben einem oder mehreren inerten Trägerstoffen und/oder Verdünndungsmitteln.

2. Arzneimittel gemäß Anspruch 1, enthaltend eine Verbindung der Formel I, in der
A und B jeweils ein Wasserstoffatom oder zusammen eine weitere Kohlenstoff-Kohlenstoff-Bindung,
$R_1$ ein Wasserstoffatom, eine Alkylgruppe mit 1 bis 4 Kohlenstoffatomen, eine in 2-Stellung durch eine Hydroxy-, Methoxy-, Methylmercapto- oder Phenylgruppe substituierte Ethylgruppe, eine gegebenenfalls durch ein Fluor-, Chlor-oder Bromatom, eine Methyl- oder Methoxygruppe substituierte Phenylgruppe, eine Cyclohexyl-, 3-Methoxy-propyl-, Allyl-, Propargyl-, Carboxymethyl-, Methoxycarbonylmethyl-, Benzyl-, Chlorbenzyl- oder Tetrahydrofurforylgruppe,

$R_2$ ein Wasserstoffatom oder eine Methylgruppe,
$R_3$ ein Wasserstoffatom, eine Methyl-, Ethyl-, Trifluormethyl- oder Phenylgruppe oder
$R_2$ und $R_3$ zusammen eine n-Propylen- oder n-Butylengruppe und
X eine Methylen-, Methyl-methylen- oder Dimethyl-methylengruppe darstellen.

3. Arzneimittel gemäß Anspruch 1, enthaltend eine Verbindung der Formel I, in der
A und B jeweils ein Wasserstoffatom oder zusammen eine weitere Kohlenstoff-Kohlenstoff-Bindung,
$R_1$ ein Wasserstoffatom, eine Alkylgruppe mit 1 bis 4 Kohlenstoffatomen oder eine in 2-Stellung durch eine Hydroxy-, Methoxy- oder Methylmercaptogruppe substituierte Ethylgruppe,
$R_2$ ein Wasserstoffatom oder eine Methylgruppe und $R_3$ ein Wasserstoffatom oder
$R_2$ ein Wasserstoffatom und $R_3$ eine Methyl- oder Ethylgruppe und
X eine Methylengruppe bedeuten.

4. Arzneimittel gemäß Anspruch 1, enthaltend
1-Methyl-7,8-dihydro-2,5(1H,6H)-chinolindion,
3-Methyl-3,4,7,8-tetrahydro-2,5(1H,6H)-chinolindion,
1-Methyl-3,4,7,8-tetrahydro-2,5(1H,6H)-chinolindion,
4-Methyl-7,8-dihydro-2,5-(1H,6H)-chinolindion,
3-Methyl-7,8-dihydro-2,5(1H,6H)-chinolindion,
1,3-Dimethyl-7,8-dihydro-2,5(1H,6H)-chinolindion,
1-Ethyl-4-methyl-7,8-dihydro-2,5(1H,6H)-chinolindion,
1-n-Butyl-4-methyl-7,8-dihydro-2,5(1H,6H)-chinolindion oder
1-Ethyl-3,4,7,8-tetrahydro-2,5(1H,6H)-chinolindion.

5. Arzneimittel gemäß Anspruch 1, enthaltend 1-Methyl-7,8-dihydro-2,5(1H,6H)-chinolindion.

6. Arzneimittel gemäß Anspruch 1, enthaltend 1-Ethyl-3,4,7,8-tetrahydro-2,5(1H,6H)-chinolindion.

7. Arzneimittel nach mindestens einem der Ansprüche 1 bis 6, dadurch gekennzeichnet, daß diese zur Bekämpfung von Schmerzzuständen, zur Bekämpfung von Fieber und Entzündungen sowie zur Bekämpfung der Symptomatik von Erkältungskrankheiten geeignet sind.

8. Verwendung der Verbindungen nach mindestens einem der Ansprüche 1 bis 6 zur Herstellung eines Arzneimittels, das zur Bekämpfung von Schmerzzuständen, zur Bekämpfung von Fieber und Entzündungen sowie zur Bekämpfung der Symptomatik von Erkältungskrankheiten geeignet ist.

9. Verfahren zur Herstellung eines Arzneimittels nach mindestens einem der Ansprüche 1 bis 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

10. Chinolin-2,5-dione der Formel

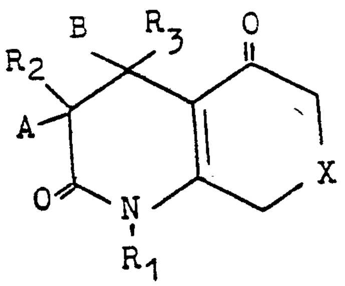

,(I)

in der,
wenn A und B zusammen eine Kohlenstoff-Kohlenstoff-Bindung darstellen,
$R_1$ ein Wasserstoffatom, eine gegebenenfalls durch eine Phenyl-, Fluorphenyl-, Chlorphenyl- oder Bromphenylgruppe, durch eine Carboxygruppe oder durch eine Alkoxycarbonylgruppe mit insgesamt 2 bis 4 Kohlenstoffatomen substituierte Alkylgruppe mit 1 bis 3 Kohlenstoffatomen, eine Alkylgruppe mit 4 bis 6 Kohlenstoffatomen, eine Cycloalkylgruppe mit 3 bis 6 Kohlenstoffatomen, eine Alkenyl- oder Alkinylgruppe mit jeweils 3 bis 5 Kohlenstoffatomen, eine in 2- oder 3-Stellung durch eine Hydroxy-, Alkoxy- oder Alkylmercaptogruppe substituierte Alkylgruppe mit 2 oder 3 Kohlenstoffatomen, in welcher der Alkoxy- oder Alkylmercaptosubstituent jeweils 1 bis 3 Kohlenstoffatome enthalten kann, eine gegebenenfalls durch ein Halogenatom, durch eine Alkyl- oder Alkoxygruppe mit jeweils 1 bis 3 Kohlenstoffatome im Alkylteil substituierte Phenylgruppe oder eine Tetrahydrofurforylgruppe,
$R_2$ eine Alkylgruppe mit 1 bis 3 Kohlenstoffatomen,
$R_3$ ein Wasserstoffatom, eine Trifluormethyl-, Phenyl- oder Alkylgruppe mit 1 bis 3 Kohlenstoffatomen oder

$R_2$ und $R_3$ zusammen eine n-Alkylengruppe mit 3 bis 5 Kohlenstoffatomen und
X eine gegebenenfalls durch eine oder zwei Alkylgruppen mit jeweils 1 bis 3 Kohlenstoffatomen substituierte Methylengruppe bedeuten oder
$R_1$ mit Ausnahme des Wasserstoffatoms, der Methyl- und Ethylgruppe die für $R_1$ vorstehend erwähnten Bedeutungen besitzt, $R_3$ und X wie vorstehend definiert sind und $R_2$ ein Wasserstoffatom darstellt,
oder, wenn A und B jeweils ein Wasserstoffatom darstellen,
$R_1$ mit Ausnahme des Wasserstoffatoms, der Methylgruppe und der Benzylgruppe die für $R_1$ vorstehend erwähnten Bedeutungen besitzt, $R_3$ und X wie vorstehend definiert sind und
$R_2$ ein Wasserstoffatom darstellt oder
$R_2$ eine Alkylgruppe mit 2 oder 3 Kohlenstoffatomen darstellt und $R_1$, $R_3$ und X wie vorstehend definiert sind.

11. Chinolin-2,5-dione der Formel I gemäß Anspruch 1, in der,
wenn A und B zusammen eine weitere Kohlenstoff-Kohlenstoff-Bindung darstellen,
$R_1$ ein Wasserstoffatom, eine Alkylgruppe mit 1 bis 4 Kohlenstoffatomen, eine in 2-Stellung durch eine Hydroxy-, Methoxy-, Methylmercapto- oder Phenylgruppe substituierte Ethylgruppe, eine gegebenenfalls durch ein Fluor-, Chlor-oder Bromatom, eine Methyl- oder Methoxygruppe substituierte Phenylgruppe, eine Cyclohexyl-, 3-Methoxy-propyl-, Allyl-, Propargyl-, Carboxymethyl-, Methoxycarbonylmethyl-, Benzyl-, Chlorbenzyl- oder Tetrahydrofurforylgruppe,
$R_2$ eine Methylgruppe,
$R_3$ ein Wasserstoffatom, eine Methyl-, Ethyl-, Trifluormethyl- oder Phenylgruppe oder
$R_2$ und $R_3$ zusammen eine n-Propylen- oder n-Butylengruppe und
X eine Methylen-, Methyl-methylen- oder Dimethyl-methylengruppe bedeuten oder
$R_1$ mit Ausnahme des Wasserstoffatoms, der Methyl- und Ethylgruppe die für $R_1$ vorstehend erwähnten Bedeutungen besitzt, $R_3$ und X wie vorstehend definiert sind und $R_2$ ein Wasserstoffatom darstellt,
oder, wenn A und B jeweils ein Wasserstoffatom darstellen,
$R_1$ mit Ausnahme des Wasserstoffatoms, der Methylgruppe, der Ethylgruppe, der Allylgruppe und der Benzylgruppe die für $R_1$ vorstehend erwähnten Bedeutungen besitzt, $R_3$ und X wie vorstehend definiert sind und $R_2$ ein Wasserstoffatom darstellt.

12. Chinolin-2,5-dione der Formel I gemäß Anspruch 1, in der, wenn A und B zusammen eine weitere Kohlenstoff-Kohlenstoff-Bindung darstellen,
$R_1$ ein Wasserstoffatom, eine Alkylgruppe mit 1 bis 4 Kohlenstoffatomen oder eine in 2-Stellung durch eine Hydroxy-, Methoxy- oder Methylmercaptogruppe substituierte Ethylgruppe,
$R_2$ eine Methylgruppe,
$R_3$ ein Wasserstoffatom und
X eine Methylengruppe oder
$R_1$ mit Ausnahme des Wasserstoffatoms, der Methyl- und Ethylgruppe die für $R_1$ vorstehend erwähnten Bedeutungen besitzt,
$R_3$ und X wie vorstehend definiert sind und $R_2$ ein Wasserstoffatom darstellt,
oder, wenn A und B jeweils ein Wasserstoffatom darstellen,
$R_1$ mit Ausnahme des Wasserstoffatoms, der Methylgruppe und der Ethylgruppe die für $R_1$ vorstehend erwähnten Bedeutungen besitzt, $R_3$ und X wie vorstehend erwähnt definiert sind und $R_2$ ein Wasserstoffatom darstellt.

13. Als Chinolin-2,5-dione der Formel I gemäß Anspruch 1:
3-Methyl-7,8-dihydro-2,5(1H,6H)-chinolindion,
1,3-Dimethyl-7,8-dihydro-2,5(1H,6H)-chinolindion,
1-Ethyl-4-methyl-7,8-dihydro-2,5(1H,6H)-chinolindion,
1-n-Butyl-4-methyl-7,8-dihydro-2,5(1H,6H)-chinolindion oder
1-Ethyl-3,4,7,8-tetrahydro-2,5(1H,6H)-chinolindion.

14. 1-Ethyl-3,4,7,8-tetrahydro-2,5(1H,6H)-chinolindion.

15. Verfahren zur Herstellung der neuen Chinolin-2,5-dione der Formel I nach mindestens einem der Ansprüche 10 bis 14, dadurch kennzeichnet, daß
a) eine Verbindung der Formel

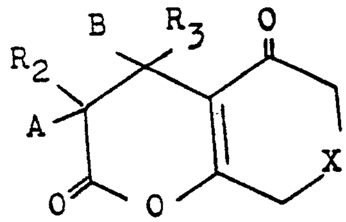

, (II)

in der
A, B, $R_2$, $R_3$ und X wie in mindestens einem der Ansprüche 10 bis 14 definiert sind, mit einem Amin der Formel

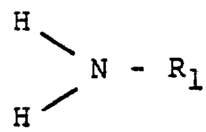

, (III)

in der
$R_1$ wie in mindestens einem der Ansprüche 10 bis 14 definiert ist, umgesetzt wird oder
b) eine Verbindung der Formel

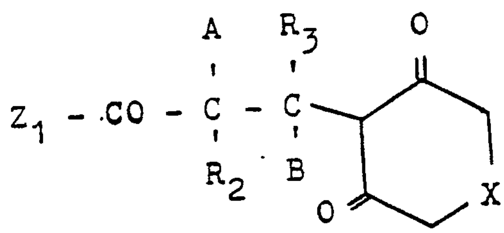

, (IV)

in der
A, B, $R_2$, $R_3$ und X wie in mindestens einem der Ansprüche 10 bis 14 definiert sind und
$Z_1$ eine nucleophile Austrittsgruppe wie eine Hydroxygruppe, eine Alkoxygruppe mit 1 bis 3 Kohlenstoffatomen oder ein Halogenatom darstellt, mit einem Amin der Formel

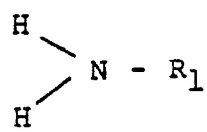

, (III)

in der
$R_1$ wie in mindestens einem der Ansprüche 10 bis 14 definiert ist, umgesetzt wird oder
c) zur Herstellung von Verbindungen der Formel I, in der $R_2$ ein Wasserstoffatom darstellt, eine Verbindung der Formel

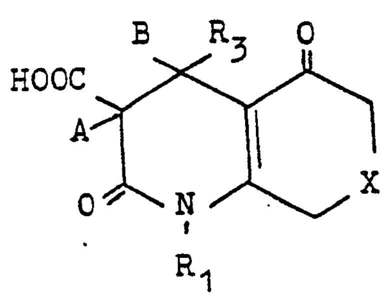

, (V)

in der
A, B, $R_1'$, $R_3$ und X wie in mindestens einem der Ansprüche 10 bis 14 definiert sind, decarboxiliert wird oder
d) zur Herstellung von Verbindungen der Formel I, in der $R_1$ mit Ausnahme des Wasserstoffatoms wie in mindestens einem der Ansprüche 10 bis 14 definiert ist, eine Verbindung der Formel

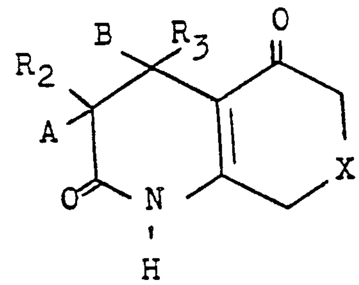

,(VI)

in der
A, B, $R_2$, $R_3$ und X wie in mindestens einem der Ansprüche 10 bis 14 definiert sind, mit einer Verbindung der Formel
Y - $R_1'$ ,(VII)
in der
$R_1'$ mit Ausnahme des Wasserstoffatoms die für $R_1$ eingangs erwähnten Bedeutungen besitzt und
Y eine nukleophile Austrittsgruppe wie ein Halogenatom, z.B. ein Chlor-, Brom- oder Jodatom, oder einen Sulfonyloxyrest, z.B. die Methansulfonyloxy- oder p-Toluolsulfonyloxygruppe, oder
Y zusammen mit einem $\beta$-Wasserstoffatom einer Alkylgruppe mit 1 bis 6 Kohlenstoffatomen des Restes $R_1'$ ein Sauerstoffatom darstellt, umgesetzt wird oder
e) zur Herstellung von Verbindungen der Formel I, in der A und B je ein Wasserstoffatom darstellen, ein Enaminoketon der Formel

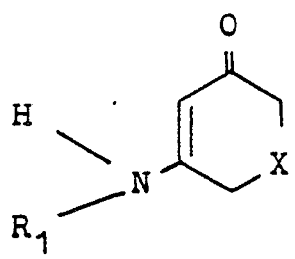

,(VIII)·

in der
$R_1$ und X wie in mindestens einem der Ansprüche 10 bis 14 definiert sind, mit einer Verbindung der Formel

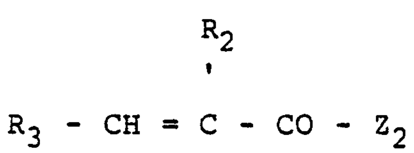

,(IX)

in der
$R_2$ und $R_3$ wie in mindestens einem der Ansprüche 10 bis 14 definiert sind und
$Z_2$ eine nukleophile Austrittsgruppe wie eine Hydroxygruppe, eine Alkoxygruppe mit 1 bis 3 Kohlenstoffatomen oder ein Halogenatom darstellt, umgesetzt wird und
gewünschtenfalls anschließend eine so erhaltene Verbindung der Formel I, sofern diese ein oder zwei asymmetrische Kohlenstoffatome enthalten, in ihre Enantiomeren, Enantiomerengemische oder Racemate, oder, sofern diese zwei asymmetrische Kohlenstoffatome enthalten, in ihre Diastereomeren bzw. Diastereomerengemische aufgetrennt werden.

16. Neue Chinolindione der Formel

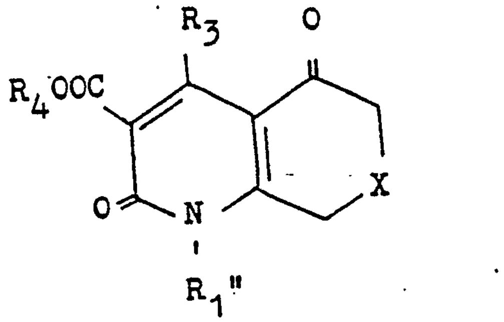

, (X)

in der
$R_3$ und X wie in mindestens einem der Ansprüche 1 bis 6 definiert sind,
$R_1''$ ein Wasserstoffatom, eine gegebenenfalls durch eine Phenyl-, Fluorphenyl-, Chlorphenyl- oder Bromphenylgruppe, durch eine Carboxygruppe oder durch eine Alkoxycarbonylgruppe mit insgesamt 2 bis 4 Kohlenstoffatomen substituierte Alkylgruppe mit 1 bis 3 Kohlenstoffatomen, eine Alkylgruppe mit 4 bis 6 Kohlenstoffatomen, eine Cycloalkylgruppe mit 3 bis 6 Kohlenstoffatomen, eine Alkenyl- oder Alkinylgruppe mit jeweils 3 bis 5 Kohlenstoffatomen, eine in 2- oder 3-Stellung durch eine Hydroxy-, Alkoxy- oder Alkylmercaptogruppe substituierte Alkylgruppe mit 2 oder 3 Kohlenstoffatomen, in welcher der Alkoxy- oder Alkylmercaptosubstituent jeweils 1 bis 3 Kohlenstoffatome enthalten kann, oder eine Tetrahydrofurforylgruppe und
$R_4$ eine Alkylgruppe mit 1 bis 5 Kohlenstoffatomen oder
$R_1''$ mit Ausnahme des Wasserstoffatoms die für $R_1''$ vorstehend erwähnten Bedeutungen besitzt und
$R_4$ ein Wasserstoffatom bedeuten.

17. Verfahren zur Herstellung der Chinolindione der Formel X gemäß Anspruch 16, dadurch gekennzeichnet, daß ein Cyclohexan-1,3-dion der Formel

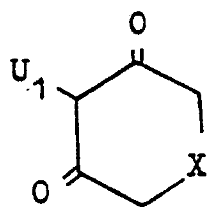

, (XI)

in der
X wie in mindestens einem der Ansprüche 1 bis 6 definiert ist, mit einem Cyanessigester der Formel

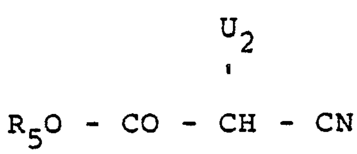

, (XII)

in der
$U_1$ ein Wasserstoffatom und $U_2$ zusammen mit dem Wasserstoffatom der benachbarten CH-Gruppe eine Gruppe der Formel

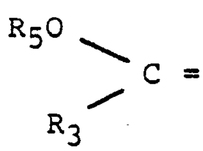

oder

$U_1$ zusammen mit dem Wasserstoffatom der benachbarten CH-Gruppe eine Gruppe der Formel

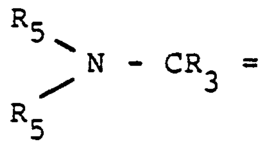 und $U_2$ ein Wasserstoffatom

bedeuten, wobei $R_3$ wie in mindestens einem der Ansprüche 1 bis 6 definiert ist und $R_5$, die gleich oder verschieden sein können, jeweils eine Alkylgruppe mit 1 bis 5 Kohlenstoffatomen darstellen, umgesetzt wird,

ein so erhaltenes Tetrahydro-cumarinon der Formel

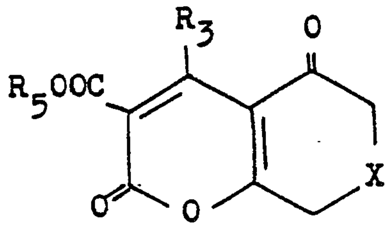

, (XIII)

in der

$R_3$ und X wie in mindestens einem der Ansprüche 1 bis 6 definiert sind und

$R_5$ wie vorstehend erwähnt definiert ist, mit einem Amin der Formel

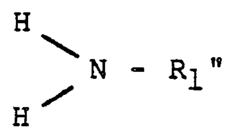

, (IIIa)

in der

$R_1''$ wie im Anspruch 16 definiert ist, umgesetzt und gewünschtenfalls anschließend ein so erhaltener Ester hydrolysiert wird.

18. Verfahren zur Herstellung von Verbindungen der Formel

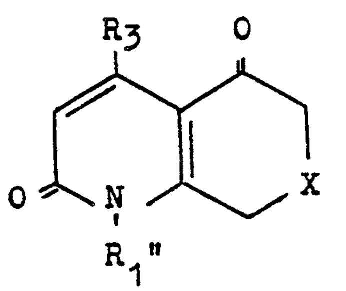

, (Ia)

in der

$R_1''$ wie im Anspruch 16 und $R_3$ und X wie in mindestens einem der Ansprüche 1 bis 6 definiert sind, dadurch gekennzeichnet, daß ein Cyclohexan-1,3-dion der Formel

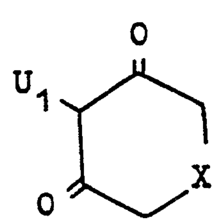

, (XI)

in der

X wie in mindestens einem der Ansprüche 1 bis 6 definiert ist, mit einem Cyanessigester der Formel

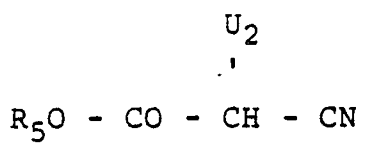

, (XII)

in der
$U_1$ ein Wasserstoffatom und $U_2$ zusammen mit dem Wasserstoffatom der benachbarten CH-Gruppe eine Gruppe der Formel

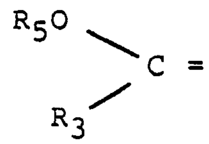

oder

$U_1$ zusammen mit dem Wasserstoffatom der benachbarten CH-Gruppe eine Gruppe der Formel

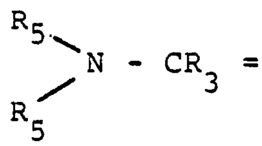

und $U_2$ ein Wasserstoffatom

bedeuten, wobei $R_3$ wie in mindestens einem der Ansprüche 1 bis 6 definiert ist und $R_5$, die gleich oder verschieden sein können, jeweils eine Alkylgruppe mit 1 bis 5 Kohlenstoffatomen darstellen, umgesetzt wird,
ein so erhaltenes Tetrahydro-cumarinon der Formel

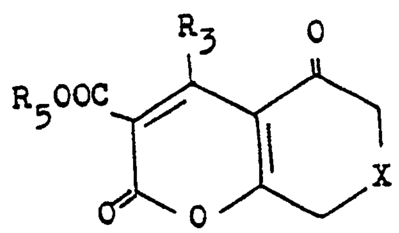

, (XIII)

in der
$R_3$ und X wie in mindestens einem der Ansprüche 1 bis 6 definiert sind und
$R_5$ wie vorstehend erwähnt definiert ist, mit einem Amin der Formel

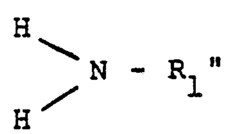

, (IIIa)

in der
$R_1''$ wie im Anspruch 16 definiert ist, umgesetzt und gewünschtenfalls anschließend ein so erhaltener Ester hydrolysiert wird und
ein so erhaltenes Chinolindion der Formel

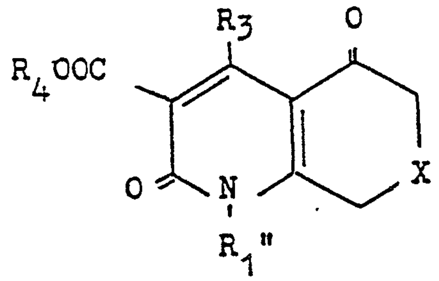

, (X)

in der

$R_1''$ und $R_4$ wie im Anspruch 16 und $R_3$ und X wie in mindestens einem der Ansprüche 1 bis 6 definiert sind, decarboxyliert wird.